(19)

Europäisches
Patentamt
European
Patent Office
Office européen
des brevets

(11)     **EP 4 474 483 A1**

(12)     **EUROPEAN PATENT APPLICATION**
published in accordance with Art. 153(4) EPC

(43) Date of publication:
**11.12.2024 Bulletin 2024/50**

(21) Application number: **23749757.3**

(22) Date of filing: **01.02.2023**

(51) International Patent Classification (IPC):
*C12Q 1/06* [(2006.01)]     *A23L 27/00* [(2016.01)]
*C12N 15/12* [(2006.01)]     *C12Q 1/6897* [(2018.01)]

(52) Cooperative Patent Classification (CPC):
**A23L 27/00; C07K 14/435; C12Q 1/06;**
**C12Q 1/6897**

(86) International application number:
**PCT/JP2023/003134**

(87) International publication number:
**WO 2023/149441 (10.08.2023 Gazette 2023/32)**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(30) Priority: **03.02.2022   JP 2022015829**

(71) Applicant: **AJINOMOTO CO., INC.**
**Chuo-ku**
**Tokyo 104-8315 (JP)**

(72) Inventors:
• **KAWATO, Yayoi**
  **Kawasaki-shi, Kanagawa 210-8681 (JP)**
• **NAKADA, Yuji**
  **Kawasaki-shi, Kanagawa 210-8681 (JP)**
• **SUGIYAMA, Shingo**
  **Kawasaki-shi, Kanagawa 210-8681 (JP)**

(74) Representative: **Strehl Schübel-Hopf & Partner**
**Maximilianstrasse 54**
**80538 München (DE)**

Remarks:
The complete document including Reference
Table(s) and the Sequence Listing(s) can be
downloaded from the EPO website

(54)     **METHOD FOR SCREENING SUBSTANCE THAT SUPPRESSES 4-METHYLACETOPHENONE ODOR, AND METHOD FOR SUPPRESSING 4-METHYL ACETOPHENONE ODOR**

(57)     A technique for suppressing 4-methylacetophenone odor is provided. Screening for a substance that suppresses 4-methylacetophenone odor is performed by using the olfactory receptor OR5P3. The substance selected by the screening is used to suppress 4-methylacetophenone odor of foods.

EP 4 474 483 A1

# EP 4 474 483 A1

**Description**

TECHNICAL FIELD

The present invention relates to a technique for suppressing 4-methylacetophenone odor. Specifically, the present invention may relate to a method for screening for a substance that suppresses 4-methylacetophenone odor and a method for suppressing 4-methylacetophenone odor.

BACKGROUND ART

Citral is an aromatic ingredient that presents a lemon flavor and is found in citrus fruits such as lemon and herbs such as lemongrass. Citral is used by, for example, adding it to food products such as beverages. Citral can decrease during, for example, heating or storage to generate a degradation odor (off-flavor). Examples of substances responsible for citral-derived degradation odor include 4-methylacetophenone and p-cresol. Therefore, it is desired to develop a technique to suppress 4-methylacetophenone odor.

For example, techniques for suppressing 4-methylacetophenone odor using various ingredients have been reported (Patent documents 1 to 3).

Methods for screening for substances that suppress specific odors using olfactory receptors have also been reported. Examples of such methods include a method for screening for a substance that suppresses 2-heptanone odor using the olfactory receptor OR5P3 (Patent document 4).

PRIOR ART DOCUMENTS

PATENT DOCUMENTS

[Patent document 1] Japanese Patent Publication No. 2015-180715
[Patent document 2] Japanese Patent Publication No. 2002-338990
[Patent document 3] Japanese Patent Publication No. 2008-280539
[Patent document 4] Japanese Patent Publication No. 2019-129772

SUMMARY OF THE INVENTION

PROBLEMS TO BE SOLVED BY THE INVENTION

An object of the present invention is to provide a technique for suppressing 4-methylacetophenone odor. Specifically, the object of the present invention may be to provide a method for screening for a substance that suppresses 4-methylacetophenone odor and a method for suppressing 4-methylacetophenone odor.

MEANS FOR SOLVING THE PROBLEMS

The inventors of the present invention diligently studied to achieve the aforementioned object, as a result, found that the olfactory receptor OR5P3 responds to 4-methylacetophenone, and that various ingredients selected by screening using inhibition of response of olfactory receptors as an indicator can suppress 4-methylacetophenone odor, and thus accomplished the present invention.

That is, the present invention can be embodied, for example, as follows.

[1] A method for screening for a substance that suppresses 4-methylacetophenone odor, comprising the following steps (A) to (C):

(A) the step of contacting an olfactory receptor with an olfactory receptor-activating substance in the presence of a test substance;
(B) the step of measuring a response of the olfactory receptor to the olfactory receptor-activating substance; and
(C) the step of identifying the test substance as a substance that suppresses 4-methylacetophenone odor on the basis of the response; and wherein

when the response is inhibited by the test substance, the test substance is identified as a substance that suppresses 4-methylacetophenone odor, and
the olfactory receptor is OR5P3.

[2] The method described above, wherein the response is activation of the olfactory receptor.

[3] The method described above, wherein the olfactory receptor is used in the form of being carried by a cell, cell membrane, artificial lipid bilayer membrane vesicle, or artificial lipid bilayer membrane.

[4] The method described above, wherein the olfactory receptor is used in the form of being carried by a cell.

[5] The method described above, wherein the cell is an animal cell.

[6] The method described above, wherein the steps (B) and (C) are carried out by the following steps (B1) and (C1), respectively:

(B1) the step of measuring degree of activation D1 of the olfactory receptor observed when the step (A) is performed; and
(C1) the step of identifying the test substance as a substance that suppresses 4-methylacetophenone odor on the basis of the degree of activation D1.

[7] The method described above, wherein the step (C1) is carried out by the following step (C2):
(C2) the step of identifying the test substance as a substance that suppresses 4-methylacetophenone odor on the basis of difference between the degree of activation D1 and degree of activation D2 of the olfactory receptor observed under a control condition.

[8] The method described above, wherein the control condition is the following condition (C2-1) or (C2-2):

(C2-1) a condition under which the olfactory receptor is contacted with the olfactory receptor-activating substance in the absence of the test substance; or
(C2-2) a condition under which the olfactory receptor is contacted with the olfactory receptor-activating substance in the presence of the test substance, and concentration of the test substance is lower than concentration of the test substance used in the step (A).

[9] The method described above, wherein the method further comprises the step of measuring the degree of activation D2.

[10] The method described above, wherein the test substance is identified as a substance that suppresses 4-methylacetophenone odor, if the degree of activation D1 is lower than the degree of activation D2.

[11] The method described above, wherein the test substance is identified as a substance that suppresses 4-methylacetophenone odor, if the ratio of the degree of activation D1 to the degree of activation D2 is lower than 60%.

[12] The method described above, wherein the response is measured by using intracellular cAMP concentration as an indicator.

[13] The method described above, wherein the intracellular cAMP concentration is measured by a reporter assay.

[14] The method described above, wherein the olfactory receptor is a human olfactory receptor.

[15] The method described above, wherein the OR5P3 is a protein described in the following (a), (b), or (c):

(a) a protein comprising the amino acid sequence shown in SEQ ID NO: 2;
(b) a protein comprising an amino acid sequence comprising substitution, deletion, insertion, and/or addition of 1 to 10 amino acid residues in the amino acid sequence shown in SEQ ID NO: 2, and having responsivity to the olfactory receptor-activating substance; or
(c) a protein comprising an amino acid sequence having an identity of 80% or higher to the amino acid sequence shown in SEQ ID NO: 2, and having responsivity to the olfactory receptor-activating substance.

[16] The method described above, wherein the olfactory receptor-activating substance is 4-methylacetophenone or 2-heptanone.

[17] The method described above, wherein the method further comprises the step of evaluating whether an identified substance that suppresses 4-methylacetophenone odor has a function to suppress 4-methylacetophenone odor.

[18] The method described above, wherein the evaluation is performed by sensory evaluation.

[19] A composition for suppressing 4-methylacetophenone odor of a food, comprising the following ingredient (A):
(A) at least one type of ingredient that inactivates the olfactory receptor OR5P3 and is selected from the group consisting of N-vanillylnonanamide, nootkatone, 2-phenylethanethiol, benzyl isothiocyanate, 2-thiophenemethanethiol, difurfuryl sulfide, 2-methylbenzenethiol, benzyl isoeugenol, 2-phenyl-2-butenal, 4-methyl-1-phenyl-2-pentanol, 2-thiophenethiol, 2,4-heptadienal, and S-(2-methyl-3-furyl) ethanethioate.

[20] A composition for producing a food, comprising the following ingredient (A):

(A) at least one type of ingredient that inactivates the olfactory receptor OR5P3 and is selected from the group consisting of N-vanillylnonanamide, nootkatone, 2-phenylethanethiol, benzyl isothiocyanate, 2-thiophenemethanethiol, difurfuryl sulfide, 2-methylbenzenethiol, benzyl isoeugenol, 2-phenyl-2-butenal, 4-methyl-1-phenyl-2-pentanol, 2-thiophenethiol, 2,4-heptadienal, and S-(2-methyl-3-furyl) ethanethioate.

[21] The composition described above, wherein the food is a food of which 4-methylacetophenone odor is suppressed.

[22] The composition described above, wherein the food comprises 4-methylacetophenone and/or an ingredient that can generate 4-methylacetophenone.

[23] The composition described above, wherein the ingredient that can generate 4-methylacetophenone is citral.

[24] A method for suppressing 4-methylacetophenone odor of a food, comprising the step of adding the following ingredient (A) to a raw material of the food:

(A) at least one type of ingredient that inactivates the olfactory receptor OR5P3 and is selected from the group consisting of N-vanillylnonanamide, nootkatone, 2-phenylethanethiol, benzyl isothiocyanate, 2-thiophenemethanethiol, difurfuryl sulfide, 2-methylbenzenethiol, benzyl isoeugenol, 2-phenyl-2-butenal, 4-methyl-1-phenyl-2-pentanol, 2-thiophenethiol, 2,4-heptadienal, and S-(2-methyl-3-furyl) ethanethioate.

[25] A method for producing a food, comprising the step of adding the following ingredient (A) to a raw material of the food:

(A) at least one type of ingredient that inactivates the olfactory receptor OR5P3 and is selected from the group consisting of N-vanillylnonanamide, nootkatone, 2-phenylethanethiol, benzyl isothiocyanate, 2-thiophenemethanethiol, difurfuryl sulfide, 2-methylbenzenethiol, benzyl isoeugenol, 2-phenyl-2-butenal, 4-methyl-1-phenyl-2-pentanol, 2-thiophenethiol, 2,4-heptadienal, and S-(2-methyl-3-furyl) ethanethioate.

[26] The method described above, wherein the food is a food of which 4-methylacetophenone odor is suppressed.

[27] The method described above, wherein the food or the raw material comprises 4-methylacetophenone and/or an ingredient that can generate 4-methylacetophenone.

[28] The method described above, wherein the ingredient that can generate 4-methylacetophenone is citral.

[29] The method described above, wherein the method comprises the step of heating the food or the raw material comprising an ingredient that can generate 4-methylacetophenone.

[30] The method described above, wherein the ingredient (A) is added so that the concentration thereof at the time of eating is 0.001 ppt (w/w) to 100 ppm (w/w).

BRIEF DESCRIPTION OF THE DRAWINGS

[Fig. 1] Drawing showing responses of various olfactory receptors to 300 $\mu$M of 4-methylacetophenone. The responses (vertical axis) are shown as values of "olfactory receptor activity".

[Fig. 2] Drawing showing responses of various olfactory receptors to 30 $\mu$M of 4-methylacetophenone. The responses (vertical axis) are shown as values of "olfactory receptor activity".

[Fig. 3] Drawing showing 4-methylacetophenone concentration dependency of the response of the olfactory receptor OR5P3 to 4-methylacetophenone (n=3). The responses (vertical axis) are shown as values of "fold increase".

MODES FOR CARRYING OUT THE INVENTION

Hereafter, the present invention will be explained in detail.

<1> Screening for substance that suppresses 4-methylacetophenone odor

The first embodiment of the method of the present invention is a method for screening for a substance that suppresses 4-methylacetophenone odor using an olfactory receptor. The first embodiment of the method of the present invention is also referred to as the "screening method of the present invention". In the screening method of the present invention, a substance that suppresses 4-methylacetophenone odor can be identified (i.e., whether a test substance is a substance that suppresses 4-methylacetophenone odor can be identified) by using an olfactory receptor. In the screening method of the present invention, specifically, a substance that suppresses 4-methylacetophenone odor can be identified (i.e., whether a test substance is a substance that suppresses 4-methylacetophenone odor can be identified) on the basis of response of the olfactory receptor to an olfactory receptor-activating substance in the presence of the test substance. In the screening method of the present invention, more specifically, a substance that suppresses 4-methylacetophenone odor can be identified (i.e., whether a test substance is a substance that suppresses 4-methylacetophenone odor can be identified) on the basis of inhibition of the response of the olfactory receptor to the olfactory receptor-activating substance by the test substance. That is, the screening method of the present invention may be, specifically, a method for screening for a substance that suppresses 4-methylacetophenone odor, comprising (A) the step of contacting an olfactory receptor with an olfactory receptor-activating substance in the presence of a test substance; (B) the step of measuring a response of the olfactory receptor to the olfactory receptor-activating substance; and (C) the step of identifying the test substance as a substance that suppresses 4-methylacetophenone odor on the basis of the response; and wherein when the response is inhibited by the test substance, the test substance is identified as a substance that suppresses 4-methylacetophenone odor.

<1-1> Substance that suppresses 4-methylacetophenone odor

The term "4-methylacetophenone odor" means the odor presented by 4-methylacetophenone.

The substance that suppresses 4-methylacetophenone odor is not particularly limited as long as it can suppress 4-methylacetophenone odor (i.e., it has a function of suppressing 4-methylacetophenone odor). The function of suppressing 4-methylacetophenone odor is also referred to as the "masking function". The substance that suppresses 4-methylacetophenone odor may consist of a single type of ingredient (i.e., pure substance) or a combination of two or more types of ingredients (i.e., mixture). The "mixture" is also referred to as the "composition". When the substance that suppresses 4-methylacetophenone odor is a mixture, the number of types and composition ratios of the ingredients of the mixture are not particularly limited. When the substance that suppresses 4-methylacetophenone odor is a mixture, each ingredient of the mixture may or may not suppresses 4-methylacetophenone odor by itself, as long as the mixture suppresses 4-methylacetophenone odor.

<1-2> Test substance

The term "test substance" means a substance used in the screening method of the present invention as a candidate for a substance that suppresses 4-methylacetophenone odor. The test substance is not particularly limited. The test substance may consist of a single type of ingredient (i.e., pure substance) or a combination of two or more types of ingredients (i.e., mixture). If the test substance is a mixture, the number of types and composition ratios of the ingredients constituting the mixture are not particularly limited. The test substance may be a known substance or a novel substance. The test substance may be a natural or artificial substance. The test substance may consist of, for example, a compound library produced by using a combinatorial chemistry technique. Examples of the test substance include, for example, alcohols, ketones, aldehydes, ethers, esters, hydrocarbons, sugars, organic acids, nucleic acids, amino acids, peptides, lipids, and various other organic or inorganic ingredients. Examples of the test substance also include, especially, existing food additives. The term "existing food additive" means a substance that has already been approved for use as a food additive. As the test substance, one type of test substance may be used, or two or more types of test substances may be used in combination. The test substance may be selected so as to contain such a substance as exemplified above, for example, existing food additives. That is, as the test substance, for example, one type of existing food additive may be used, two or more types of food additives may be used in combination, or one or more types of food additives and one or more types of other ingredients may be used in combination. If the screening method of the present invention is performed by contacting two or more types of ingredients together with an olfactory receptor, it can be identified whether the combination of those ingredients as a whole is a substance that suppresses 4-methylacetophenone odor. Examples of "contacting two or more types of ingredients together with an olfactory receptor" include contacting a test substance that is a mixture with an olfactory receptor, and contacting two or more types of test substances together with an olfactory receptor.

<1-3> Olfactory receptor-activating substance

The term "olfactory receptor-activating substance" means a substance that activates an olfactory receptor. The olfactory receptor-activating substance is not particularly limited as long as it can activate an olfactory receptor. The olfactory receptor-activating substance may consist of a single type of ingredient (i.e., pure substance) or a combination of two or more types of ingredients (i.e., mixture). If the olfactory receptor-activating substance is a mixture, each ingredient of the mixture may or may not activate an olfactory receptor by itself, as long as the mixture activates the olfactory receptor. If the olfactory receptor-activating substance is a mixture, the number of types and composition ratios of the ingredients constituting the mixture are not particularly limited. The olfactory receptor-activating substance may be a known substance or a novel substance. The olfactory receptor-activating substances may be a natural or artificial substance. The olfactory receptor-activating substance may or may not be known to be able to activate an olfactory receptor. As the olfactory receptor-activating substance, for example, a substance that can activate an olfactory receptor may be selected from such test substances as exemplified above, and used. Examples of the olfactory receptor-activating substance include 4-methylacetophenone. Examples of the olfactory receptor-activating substance also include 2-heptanone (Japanese Patent Publication No. 2019-129772).

<1-4> Olfactory receptor

As the olfactory receptor, OR5P3 is used. That is, the term "olfactory receptor" means OR5P3, unless otherwise noted. A gene encoding an olfactory receptor is also referred to as a "receptor gene".

The term "OR5P3" means an olfactory receptor classified as olfactory receptor family 5 subfamily S member 3. The gene encoding OR5P3 is also referred to as the "OR5P3 gene". OR5P3 has responsivity to an olfactory receptor-activating substance such as 4-methylacetophenone and 2-heptanone. As OR5P3, one type of OR5P3 may be used, or a combination of two or more types of OR5P3 may be used.

The expression "olfactory receptor has responsivity to an olfactory receptor-activating substance" may mean that the olfactory receptor is activated by the olfactory receptor-activating substance.

Examples of the olfactory receptor gene and olfactory receptor include olfactory receptor genes and olfactory receptors of various organisms. Examples of the organisms include, for example, animals such as mammals. Specific examples of mammals include, for example, *Homo sapiens* (human), *Mus musculus* (mouse), *Rattus norvegicus* (rat), *Canis lupus familiaris* (dog), *Felis catus* (cat), *Bos taurus* (cattle), *Sus scrofa* (pig), *Pan troglodytes* (chimpanzee), *Macaca fascicularis* (crab-eating monkey), and *Equus caballus* (horse). Examples of animals such as mammals include, in particular, humans. Nucleotide sequences of olfactory receptor genes and amino acid sequences of olfactory receptors of various organisms can be obtained from public databases, for example, NCBI and Ensembl. The nucleotide sequence of the human OR5P3 gene and the amino acid sequence of the human OR5P3 are shown in SEQ ID NOS: 1 and 2, respectively.

That is, the olfactory receptor gene may be, for example, a gene having a known or natural nucleotide sequence of any of such olfactory receptor genes as described above (e.g., the nucleotide sequences of the olfactory receptor genes of the organisms exemplified above registered in NCBI or the nucleotide sequence of SEQ ID NO: 1). The olfactory receptor may be, for example, a protein having a known or natural amino acid sequence of any of such olfactory receptors as described above (e.g., the amino acid sequences of the olfactory receptors of the organisms exemplified above registered in NCBI or the amino acid sequence of SEQ ID NO: 2). The expression "a gene has a nucleotide sequence" means that the gene contains the nucleotide sequence unless otherwise specified, and also encompasses the case where the gene consists of the nucleotide sequence. The expression "a protein has an amino acid sequence" means that the protein contains the amino acid sequence, unless otherwise specified, and also encompasses the case where the protein consists of the amino acid sequence.

The olfactory receptor may be, for example, a chimeric protein of two or more types of olfactory receptors of different origins. In other words, OR5P3 shall also encompass, for example, chimeric proteins of two or more types of OR5P3 of different origins. Such a chimeric protein is also referred to as a "chimeric olfactory receptor". In other words, the term "chimeric olfactory receptor" means a protein having a chimeric sequence of olfactory receptors (i.e., a protein having a chimeric sequence of two or more types of olfactory receptors of different origins). The term "chimeric sequence of olfactory receptors" means a chimeric sequence of amino acid sequences of olfactory receptors (i.e., chimeric sequence of amino acid sequences of two or more olfactory receptors of different origins). The term "chimeric sequence of olfactory receptor" specifically means an amino acid sequence of an olfactory receptor of which partial sequence or sequences are replaced with a partial sequence or sequences of one or more types of olfactory receptors of other origins. Substitution of amino acid sequence in construction of a chimeric olfactory receptor can be carried out between corresponding sites in the amino acid sequences of the olfactory receptors. The term "corresponding sites in the amino acid sequences of the olfactory receptors" means sites that locate at corresponding positions in the alignment of the amino acid sequences of those olfactory receptors. Examples of the chimeric olfactory receptor include, for example, chimeric proteins of olfactory receptors of the organisms exemplified above (i.e., chimeric proteins of olfactory receptors of two or more types of organisms selected from the organisms exemplified above). Specific examples of the chimeric olfactory receptor include, for example, chimeric olfactory receptors of mammals (i.e., chimeric proteins of olfactory receptors of two or more mammalian species). That is, the olfactory receptor may be, for example, a protein having a chimeric sequence of the amino acid sequences of the olfactory receptors of the organisms exemplified above (specifically, a chimeric sequence of the amino acid sequences of the olfactory receptors of two or more types of organisms selected from the organisms exemplified above). As the chimeric olfactory receptor, one that has responsivity to an olfactory receptor-activating substance such as 4-methylacetophenone or 2-heptanone can be selected.

The number of types of organisms from which the olfactory receptors constituting the chimeric olfactory receptor are derived is not particularly limited. The number of types of organisms from which the olfactory receptors constituting the chimeric olfactory receptor are derived may be 2, 3, or larger.

The composition ratio of each olfactory receptor derived from each organism in the chimeric olfactory receptor is not particularly limited. The composition ratio of the olfactory receptor derived from each organism can be set as appropriate so long as the total of the composition ratios of the olfactory receptors derived from the organisms constituting the chimeric olfactory receptor does not exceed 100%. The composition ratio of olfactory receptor derived from each organism may be, for example, 1% or higher, 3% or higher, 5% or higher, 10% or higher, 20% or higher, 40% or higher, 50% or higher, 60% or higher, 70% or higher, 80% or higher, 90% or higher, 95% or higher, 97% or higher, or 99% or higher, and may be 99% or lower, 97% or lower, 95% or lower, 90% or lower, 80% or lower, 70% or lower, 60% or lower, 50% or lower, 40% or lower, 30% or lower, 20% or lower, 10% or lower, 5% or lower, 3% or lower, or 1% or lower, or may be in a range defined by any non-contradictory combination of these exemplary minimum and maximum composition ratios. The term "composition ratio of olfactory receptor derived from each organism" means the ratio of the number of amino acid residues of the olfactory receptor derived from each organism to the total number of amino acid residues constituting the chimeric olfactory receptor. If certain amino acid residues constituting the chimeric olfactory receptor correspond to a conserved sequence of the olfactory receptors derived from the organisms that constitute the chimeric olfactory receptor, the amino acid residues may be considered to be derived from any of the organisms.

The mode of distribution of the olfactory receptors derived from the respective organisms in the chimeric olfactory receptor is not particularly limited. In the chimeric olfactory receptor, the olfactory receptor derived from each organism may be located at one location as a cluster or distributed in two or more locations. For example, if a chimeric olfactory receptor is designed by replacing an internal amino acid sequence of an olfactory receptor derived from one organism (olfactory receptor A) with that of an olfactory receptor derived from another organism (olfactory receptor B), the amino acid sequence of the olfactory receptor A will remain while distributed on the N- and C-terminal sides of the chimeric olfactory receptor.

Similarly, examples of the olfactory receptor gene include a chimeric olfactory receptor gene. The description for the chimeric olfactory receptor can be applied to the chimeric olfactory receptor gene.

The olfactory receptor gene may be a variant of any of the olfactory receptor genes exemplified above (e.g., a variant of a gene having a nucleotide sequence of any of the olfactory receptor genes of the organisms exemplified above or a chimeric sequence thereof), as long as the original function is maintained. Similarly, the olfactory receptor may be a variant of any of the olfactory receptors exemplified above (e.g., a variant of a protein having an amino acid sequence of any of the olfactory receptors of the organism exemplified above or a chimeric sequence thereof), as long as the original function is maintained. Such a variant in which the original function is maintained may be referred to as a "conservative variant". The term "OR5P3 gene" shall encompass the OR5P3 genes exemplified above as well as conservative variants thereof. Similarly, the term "OR5P3" shall encompass the OR5P3 exemplified above as well as conservative variants thereof. Examples of the conservative variants include, for example, homologues and artificial modifications of the olfactory receptor genes and olfactory receptors exemplified above.

The olfactory receptor gene specified by a species from which the gene has been derived is not limited to the olfactory receptor gene itself found in the species, but also encompasses genes having the nucleotide sequence of the olfactory receptor gene found in the species and conservative variants thereof. Similarly, the olfactory receptor specified by the species from which the olfactory receptor has been derived is not limited to the olfactory receptor itself found in that species, but also encompasses proteins having the amino acid sequence of the olfactory receptor found in that species and conservative variants thereof. Such conservative variants may or may not be found in the species. For example, the term "mammalian olfactory receptor" shall encompass proteins having any of amino acid sequences of olfactory receptors found in mammals and conservative variants thereof. Further, for example, the term "mammalian chimeric olfactory receptor" shall encompass proteins having a chimeric sequence of any of amino acid sequences of olfactory receptors found in mammals and conservative variants thereof. In other words, the olfactory receptor constituting a "mammalian chimeric olfactory receptor" is not limited to the olfactory receptors found in mammals themselves, but may also encompass conservative variants thereof.

The expression "original function is maintained" means that the variant of gene or protein has a function (activity or property) that corresponds to the function (activity or property) of the original gene or protein. The expression "original function is maintained" used for a gene means that the variant of the gene encodes a protein that maintains the original function. That is, the expression "original function is maintained" used for each olfactory receptor gene means that the variant of the gene encodes an olfactory receptor that has responsivity to an olfactory receptor-activating substance such as 4-methylacetophenone and 2-heptanone. The expression "original function is maintained" used for each olfactory receptor may mean that the variant of the olfactory receptor has responsivity to an olfactory receptor-activating substance such as 4-methylacetophenone or 2-heptanone.

Whether an olfactory receptor has responsivity to an olfactory receptor-activating substance can be confirmed by, for example, measuring a response (e.g., activation) of the olfactory receptor when the olfactory receptor is contacted with the olfactory receptor-activating substance.

Hereafter, conservative variants will be exemplified..

Homologues of the olfactory receptor gene or homologues of the olfactory receptor can be easily obtained from public databases by, for example, BLAST search or FASTA search using any of the nucleotide sequences of the olfactory receptor genes exemplified above or the amino acid sequences of the olfactory receptors exemplified above as a query sequence. Homologues of the olfactory receptor gene can be obtained by, for example, PCR using any of the chromosomes of various organisms as the template and oligonucleotides prepared on the basis of any of the nucleotide sequences of these known olfactory receptor genes as primers.

The olfactory receptor gene may be a gene encoding a protein having an amino acid sequence in which one or several amino acids at one or several positions are substituted, deleted, inserted, and/or added in any of the amino acid sequences mentioned above (e.g., the amino acid sequences of the olfactory receptors of the organisms exemplified above and chimeric sequences thereof) , as long as the original function is maintained. For example, the encoded protein may have an extended or shortened N- and/or C-terminus. The number meant by the term "one or several" mentioned above means, depending on the positions and types of amino acid residues in the three-dimensional structure of the protein, specifically, for example, 1 to 50, 1 to 40, or 1 to 30, preferably 1 to 20, more preferably 1 to 10, further preferably 1 to 5, especially preferably 1 to 3.

The substitution, deletion, insertion, and/or addition of one or several amino acids mentioned above is a conservative mutation that maintains the normal function of the protein. Typical examples of the conservative mutation include conservative substitutions. Conservative substitutions are such mutations that mutual substitution occurs among Phe, Trp, and Tyr when the substitution site is an aromatic amino acid, among Leu, Ile, and Val when the substitution site is a hydrophobic amino acid, between Gln and Asn when the substitution site is a polar amino acid, among Lys, Arg, and His when the substitution site is a basic amino acid, between Asp and Glu when the substitution site is an acidic amino acid, and between Ser and Thr when the substitution site is an amino acid with hydroxyl group. Specific examples of substitutions considered conservative substitutions include substitution of Ser or Thr for Ala, substitution of Gln, His or Lys for Arg, substitution of Glu, Gln, Lys, His or Asp for Asn, substitution of Asn, Glu or Gln for Asp, substitution of Ser or Ala for Cys, substitution of Asn, Glu, Lys, His, Asp or Arg for Gln, substitution of Gly, Asn, Gln, Lys or Asp for Glu, substitution of Pro for Gly, substitution of Asn, Lys, Gln, Arg or Tyr for His, substitution of Leu, Met, Val or Phe for Ile, substitution of Ile, Met, Val or Phe for Leu, substitution of Asn, Glu, Gln, His or Arg for Lys, substitution of Ile, Leu, Val or Phe for Met, substitution of Trp, Tyr, Met, Ile or Leu for Phe, substitution of Thr or Ala for Ser, substitution of Ser or Ala for Thr, substitution of Phe or Tyr for Trp, substitution of His, Phe or Trp for Tyr, and substitution of Met, Ile or Leu for Val. The aforementioned substitution, deletion, insertion, or addition of amino acids may be those resulting from a naturally-occurring mutation (mutant or variant) due to an individual difference, difference of species, or the like of the organism from which the gene is derived.

The olfactory receptor gene may also be a gene encoding a protein having an amino acid sequence showing an identity of, for example, 50% or higher, 65% or higher, or 80% or higher, preferably 90% or higher, more preferably 95% or higher, further preferably 97% or higher, especially preferably 99% or higher, to the entire sequence of any of the amino acid sequences mentioned above, as long as the original function is maintained.

The olfactory receptor gene may also be a gene, such as DNA, that hybridizes to a probe that can be prepared from any of the nucleotide sequences mentioned above (e.g., the nucleotide sequences of olfactory receptor genes of organisms exemplified above or chimeric sequences thereof), such as a sequence complementary to the entire sequence of any of the aforementioned nucleotide sequences or a part thereof, under stringent conditions, as long as the original function is maintained. The term "stringent conditions" refers to conditions under which a so-called specific hybrid is formed, and a non-specific hybrid is not formed. Examples of the stringent conditions include those under which DNAs having a high identity to each other, for example, DNAs having an identity to each other of 50% or higher, 65% or higher, or 80% or higher, preferably 90% or higher, more preferably 95% or higher, further preferably 97% or higher, particularly preferably 99% or higher, hybridize to each other, and DNAs having an identity lower than the above do not hybridize to each other, or the washing conditions of usual Southern hybridization, i.e., washing once, preferably 2 or 3 times, at salt concentrations and temperature corresponding to 1 x SSC and 0.1% SDS at 60°C, preferably 0.1 x SSC and 0.1% SDS at 60°C, more preferably 0.1 x SSC and 0.1% SDS at 68°C.

As described above, the probe used for the hybridization mentioned above may be a part of a complementary sequence of the gene. Such a probe can be prepared by PCR using oligonucleotides prepared on the basis of a known gene sequence as primers and a DNA fragment containing the gene described above as a template. For example, a DNA fragment of about 300 bp in length can be used as the probe. When a DNA fragment having a length of about 300 bp is used as the probe, the washing conditions of the hybridization can be, for example, 50°C, 2 x SSC, and 0.1% SDS.

In addition, since the degeneracy of codons varies from host to host, the olfactory receptor gene may have replacement of any codon with an equivalent codon. In other words, the olfactory receptor gene may be, for example, a variant of any of the olfactory receptor genes exemplified above occurring due to the degeneracy of codons. For example, the olfactory receptor gene may be modified to have optimal codons according to the codon usage frequency of the host used.

For the present invention, the term "gene" is not limited to DNA, but may encompass any polynucleotide as long as it encodes a desired protein. In other words, the term "olfactory receptor gene" may mean any polynucleotide that encodes an olfactory receptor. The olfactory receptor gene may be DNA, RNA, or a combination thereof. The olfactory receptor gene may be single-stranded or double-stranded. The olfactory receptor gene may be single-stranded DNA or single-stranded RNA. The olfactory receptor gene may be double-stranded DNA, double-stranded RNA, or a hybrid strand consisting of DNA and RNA strands. The olfactory receptor gene may contain both DNA and RNA residues in a single polynucleotide strand. If the olfactory receptor gene contains RNA, the description for DNA such as the nucleotide sequences exemplified above may be read as appropriate for RNA. The olfactory receptor gene may or may not contain an intron. The type of the olfactory receptor gene may be appropriately selected according to various conditions, such as the mode of use thereof.

The term "identity" between amino acid sequences means an identity between amino acid sequences calculated with blastp using the default settings of Scoring Parameters (Matrix, BLOSUM62; Gap Costs, Existence=11 and Extension=1; and Compositional Adjustments, Conditional compositional score matrix adjustment). The term "identity" between nucleotide sequences means an identity between nucleotide sequences calculated with blastn using the default settings of Scoring Parameters (Match/Mismatch Scores=1, -2; and Gap Costs=Linear).

The olfactory receptor may contain another amino acid sequence in addition to any of such amino acid sequences of olfactory receptor as exemplified above. That is, the olfactory receptor may be a fusion protein of any of such amino acid sequences of olfactory receptor as exemplified above and another sequence. The other amino acid sequence is not particularly limited as long as the olfactory receptor has responsivity to an olfactory receptor-activating substance. Examples of the other amino acid sequence include, for example, tag sequences such as His tags and V5 epitope tags. The other amino acid sequence may be linked to, for example, the N-terminus or C-terminus, or both, of the olfactory receptor.

The olfactory receptor can be used in any form that can be used for screening for substances that suppress 4-methylacetophenone odor. That is, specifically, the olfactory receptor can be used in any form as long as the olfactory receptor can be contacted with a test substance and the olfactory receptor has responsivity to an olfactory receptor-activating substance. The mode of use of the olfactory receptor can be appropriately set according to various conditions, such as the manner in which the screening method of the present invention is performed.

The olfactory receptor may be used, for example, in a form isolated to a desired degree, such as a purified or roughly purified product, or may be used in a form contained in a material. Specifically, the olfactory receptor may be used in the form of, for example, being carried by a structure. Examples of the structure include, for example, cell, cell membrane, artificial lipid bilayer membrane vesicle, and artificial lipid bilayer membrane. Examples of the structure include, especially, cell. In other words, the olfactory receptor can be used in the form of a structure having (carrying) the olfactory receptor, such as, for example, cell having the olfactory receptor, cell membrane having the olfactory receptor, artificial lipid bilayer membrane vesicle having the olfactory receptor, or artificial lipid bilayer membrane having the olfactory receptor. These structures having the olfactory receptor may also be used, for example, in a form isolated to a desired degree or in a form contained in a material. The olfactory receptor may also constitute a part of a device. That is, the olfactory receptor may be used in the form of, for example, a device having the olfactory receptor. Examples of the device having the olfactory receptor include a device on which the olfactory receptor is immobilized and a device having a structure (such as lipid bilayer membrane) carrying the olfactory receptor. Examples of the device having a lipid bilayer membrane include, for example, chips having arrayed lipid bilayer membranes (WO2005/000558; Watanabe R. et al., Arrayed lipid bilayer chambers allow single-molecule analysis of membrane transporter activity. Nat Commun. 2014 Jul 24;5:4519; and Kamiya K. et al., Preparation of artificial cell membrane and single ion channel measurement. Electrochemistry, 83, 1096-1100 (2015)) and ion channel measurement devices having lipid bilayer membranes prepared by droplet contact method (Kawano R. et al., Automated Parallel Recordings of Topologically Identified Single Ion Channels, Scientific Reports, 3, No. 1995 (2013)). All of these forms of olfactory receptor are included in the scope of the olfactory receptor used in the screening method of the present invention.

The olfactory receptor can be produced by, for example, expressing the olfactory receptor gene. Expression of the olfactory receptor gene can be carried out by, for example, using cells or a cell-free protein synthesis system. For the expression of the olfactory receptor gene using cells, the description for cells having olfactory receptor mentioned later can be referred to. The expressed olfactory receptor can be obtained in any of the forms described above, as appropriate, and used in the screening method of the present invention.

A cell having the olfactory receptor is also referred to as the "cell of the present invention". The olfactory receptor can, for example, localize and function in the cell membrane. Therefore, the cell of the present invention may have the olfactory receptor, for example, in the cell membrane.

The olfactory receptor is expressed from the olfactory receptor gene. Therefore, the cell of the present invention has the olfactory receptor gene. Specifically, the cell of the present invention has the olfactory receptor gene in such a manner that the olfactory receptor gene can be expressed. It is sufficient that the cell of the present invention has the olfactory receptor gene until it expresses the olfactory receptor. That is, the cell of the present invention may or may not have the olfactory receptor gene after the expression of the olfactory receptor. In other words, the cell of the present invention is a cell expressing the olfactory receptor gene and also a cell expressing the olfactory receptor. The terms "expression of the olfactory receptor gene" and "expression of the olfactory receptor" may be used synonymously.

The cell of the present invention may have one copy of the olfactory receptor gene or two or more copies of the olfactory receptor gene.

The cell of the present invention may inherently have the olfactory receptor gene or may have been modified to have the olfactory receptor gene.

Examples of such a cell that inherently has the olfactory receptor gene include cells of such organisms as described above from which the olfactory receptor gene is derived, e.g., taste cells of mammals such as humans. A cell that inherently has an olfactory receptor gene can be obtained, for example, from organisms or tissues that contain such a cell.

Examples of such a cell that has been modified to have an olfactory receptor gene include cells into which an olfactory receptor gene has been introduced.

The cell of the present invention and cells used to obtain it (e.g., cells into which an olfactory receptor gene is to be or has been introduced) are also collectively referred to as "host cell".

The host cell is not particularly limited so long as it can express a functional olfactory receptor and thus can be used for screening for substances that suppress 4-methylacetophenone odor. Examples of the host cell include bacterial cells, fungal cells, plant cells, insect cells, and animal cells. Preferred examples of the host cell include eukaryotic cells such as fungal cells, plant cells, insect cells, and animal cells. More preferred examples of the host cell include animal cells. Examples of the animal include, for example, mammals, birds, and amphibians. Examples of mammals include, for example, rodents and primates. Examples of rodents include Chinese hamster, hamster, mouse, rat, and guinea pig. Examples of primates include human, monkey, and chimpanzee. Examples of birds include, for example, chicken. Examples of amphibians include, for example, African clawed frog. The tissue or cell from which the host cell is derived is not particularly limited. Examples of tissue or cell from which host cell is derived include, for example, ovary, kidney, adrenal gland, lingual epithelium, olfactory epithelium, pineal gland, thyroid gland, and melanocyte. Examples of Chinese hamster cell include, for example, those of Chinese hamster ovary-derived cell line (CHO). Specific examples of CHO include, for example, CHO-DG44 and CHO-K1. Examples of human cell include, for example, those of human embryonic kidney cell-derived cell line (HEK). Specific examples of HEK include, for example, HEK293 and HEK293T. Examples of monkey cell include, for example, those of African green monkey kidney cell-derived cell line (COS). Specific examples of COS include, for example, COS-1. Examples of African clawed frog cell include, for example, African clawed frog oocytes. Examples of insect cells include, for example, *Spodoptera frugiperda*-derived cells such as Sf9, Sf21, and SF+, and *Trichoplusia* ni-derived cells such as High-Five. The host cell may be an independent individual cell (e.g., free cell) or may form an aggregate such as tissue.

The olfactory receptor gene can be obtained by cloning from an organism that has the olfactory receptor gene. A nucleic acid such as genomic DNA or cDNA containing the gene can be used for the cloning. The olfactory receptor gene can also be obtained by chemical synthesis (Gene, 60(1), 115-127 (1987)).

The obtained olfactory receptor gene can be used as it is or after modified as appropriate. That is, the olfactory receptor gene can be modified to obtain a variant thereof. The gene can be modified by known methods. For example, a site-specific mutagenesis method can be used to introduce a desired mutation at a target site of DNA. That is, for example, a coding region of a gene can be modified by the site-specific mutagenesis method so that the encoded protein contains substitution, deletion, insertion, and/or addition of amino acid residues at a specific site. Examples of the site-specific mutagenesis method include methods using PCR (Higuchi, R., 61, in PCR technology, Erlich, H.A. Eds., Stockton press (1989); and Carter, P., Meth. in Enzymol. 154, 382 (1987)) and methods using phage (Kramer, W. and Frits, H.J., Meth. in Enzymol., 154, 350 (1987); and Kunkel, T.A., et al. Meth. in Enzymol., 154, 367 (1987)). A variant of the olfactory receptor gene may also be directly obtained by chemical synthesis.

The form for introducing the olfactory receptor gene into the host cell is not particularly limited. It is sufficient that the olfactory receptor gene is retained in the host cell so that it can be expressed. Specifically, for example, when the olfactory receptor gene is introduced in a form that requires transcription, such as DNA, the olfactory receptor gene may be retained in the host cell in such a manner that it can be expressed under the control of a promoter that functions in the host cell. In the host cell, the olfactory receptor gene may be present outside the chromosome or has been introduced into the chromosome. When two or more genes are introduced, it is sufficient that each gene is retained in the host cell in such a manner that it can be expressed.

The promoter for expression of the olfactory receptor gene is not particularly limited as long as it functions in the host cell. The term "promoter that functions in the host cell" means a promoter that shows a promoter activity in the host cell. The promoter may be a promoter derived from the host cell or a promoter of heterologous origin. The promoter may be a promoter unique to the olfactory receptor gene or a promoter of another gene. The promoter may be a stronger promoter than the promoter unique to the olfactory receptor gene. Examples of promoter that functions in animal cells include, for example, SV40 promoter, EF1a promoter, RSV promoter, CMV promoter, and SRalpha promoter. As the promoter, highly active forms of conventional promoters may be obtained by using various reporter genes, and used. Examples of method for evaluating promoter strength and strong promoter are described in the paper of Goldstein et al. (Prokaryotic promoters in biotechnology. Biotechnol. Annu. Rev., 1, 105-128 (1995)), and so forth.

The olfactory receptor gene can be introduced into a host cell by using, for example, a vector containing the gene. The vector containing the olfactory receptor gene is also referred to as the "expression vector of the olfactory receptor gene". The expression vector of the olfactory receptor gene can be constructed by, for example, ligating a DNA fragment containing the olfactory receptor gene with a vector. By introducing the expression vector of the olfactory receptor gene into a host cell, the gene can be introduced into the host cell. The vector may have a marker, such as a drug resistance gene. The vector may also have an expression regulatory sequence such as promoter for expressing the inserted gene. The vectors can be appropriately selected depending on various conditions, such as the type of the host cell and the mode of the introduction of the olfactory receptor gene. For example, examples of vector that can be used for gene transfer into animal cells include plasmid vectors and viral vectors. Examples of viral vectors include, for example, retrovirus vectors and adenovirus vectors. Examples of plasmid vectors include, for example, pcDNA series vectors (pcDNA3.1, etc., Thermo Fisher Scientific), pBApo-CMV series vectors (Takara Bio), and pCI-neo (Promega). Depending on the type and configuration of the vector, the vector can be incorporated into the host cell chromosome, autonomously replicate outside the host cell chromosome, or be temporarily retained outside the host cell chromosome. For example, a vector having a viral replication origin, such the SV40 replication origin, can autonomously replicate outside the chromosomes of animal cell. Specifically, for example, the pcDNA series vectors have the SV40 replication origin, and can autonomously replicate outside the chromosome in host cells that express the SV40 large T antigen (such as COS-1 and HEK293T).

The olfactory receptor gene can also be introduced into a host cell by, for example, introducing a nucleic acid fragment containing the gene into the host cell. A nucleic acid fragment containing the olfactory receptor gene is also referred to as the "olfactory receptor gene fragment". Examples of such a fragment include linear DNA and linear RNA. Examples of linear RNA include mRNA and cRNA.

The method for introducing a nucleic acid such as vectors and nucleic acid fragments into a host cell can be selected according to various conditions such as the type of the host cell. Examples of the method for introducing a nucleic acid such as vectors and nucleic acid fragments into a host cell such as an animal cell include, for example, the DEAE-dextran method, calcium phosphate method, lipofection method, electroporation method, and microinjection method. If the vector is a virus vector, it can be introduced into the host cell by infecting the host cell with the vector (virus).

A cell that inherently has the olfactory receptor gene may also be modified so that the expression of the olfactory receptor gene increases before use. The expression "expression of a gene increases" means that expression amount of the gene per cell increases compared with an unmodified cell. The term "unmodified cell" used here means a control cell that has not been modified so that the expression of the target gene increases. Examples of unmodified cell include wild-type cells and original cells before modification. Examples of techniques for increasing expression of the olfactory receptor gene include increasing copy number of the olfactory receptor gene and improving transcription efficiency or translation efficiency of the olfactory receptor gene. Increasing the copy number of the olfactory receptor gene can be achieved by introducing the olfactory receptor gene into the host cell. Introduction of the olfactory receptor gene can be performed as described above. The olfactory receptor gene to be introduced may be of host cell origin or of heterologous origin. Improvement of transcription efficiency or translation efficiency of the olfactory receptor gene can be achieved by modifying an expression regulatory sequence of the gene such as promoter. For example, improvement of the transcription efficiency of the olfactory receptor gene can be achieved by replacing the promoter of the olfactory receptor gene with a stronger promoter.

The cell of the present invention may have any other property as long as it can be used to screen for substances that suppress 4-methylacetophenone odor. Examples of such a property include, for example, properties useful for measuring a response of olfactory receptor to an olfactory receptor-activating substance. The description for the properties of the cell of the present invention can be applied to the cases of using olfactory receptors in other forms. Examples of the cases of using olfactory receptor in other forms include the cases of using artificial lipid bilayer membrane vesicle, cell membrane, or artificial lipid bilayer membrane having the olfactory receptor.

The cell of the present invention may or may not have, for example, an olfactory receptor other than the selected olfactory receptor (also referred to as the "other olfactory receptor"). It may be desirable that the cell of the present invention has no other olfactory receptors. Examples of cell having no other olfactory receptors include cells that do not have any gene encoding another olfactory receptor or cells that have a gene encoding another olfactory receptor but do not express the gene. The cell of the present invention may be, for example, a cell inherently devoid of other olfactory receptors or may be modified to be devoid of other olfactory receptors. Such a modification of a cell that the cell does not have other olfactory receptors can be achieved by, for example, knocking out any gene encoding another olfactory receptor.

The cell of the present invention may also have, for example, a protein involved in signal transduction. In other words, the cell of the present invention may have a gene encoding a protein involved in signal transduction. Examples of such a protein involved in signal transduction include, for example, G proteins (such as Golf), G protein activators (such as Ric8B), adenylate cyclase, and calcium channel. Examples of Golf include, for example, animal Golf such as human Golf (GenBank accession No. NP_892023). Examples of Ric8B includes, for example, animal Ric8B such as rat Ric8B (GenBank accession No. NP_783188). The cell of the present invention may also have, for example, a component appropriate for parameters to be measured. Examples of such a component include probes such as those for calcium indicators and reporter genes such as luciferase gene. When a probe for a calcium indicator or the like is expressed from a gene, the cell of the present invention may have a gene encoding such a probe.

The cell of the present invention may also have, for example, a protein that promotes membrane expression of the olfactory receptor. In other words, the cell of the present invention may have a gene encoding such a protein. Examples of such a protein include, for example, RTP1s (Zhuang H. and Matsunami H., J. Biol. Chem., 282, 15284-15293 (2007)). Examples of RTP1s include, for example, animal RTP1s such as human RTP1s (GenBank accession No. AAT70680), mouse RTP1s (GenBank accession No. ABU23737) and bat RTP1s (amino acid sequence from the methionine residue at position 37 to the C-terminus of GenBank accession No. XP_006765914). The amino acid sequence of mouse RTP1s has an identity of 93.3% to the amino acid sequence of human RTP1s. The amino acid sequence of bat RTP1s (the partial sequence mentioned above) has an identity of 90.7% to the amino acid sequence of human RTP1s.

The cell of the present invention may be a cell that inherently has such properties as exemplified above, or may be a cell modified to have such properties as exemplified above. For the modification of cell, the descriptions for modification of cell concerning the olfactory receptor gene, such as introduction of the olfactory receptor gene, can be applied. The genes exemplified above may be a gene derived from the host cell or a gene derived from a different species. The genes exemplified above may or may not be of the same origin as the olfactory receptor gene. When two or more genes are introduced, it is sufficient that each gene is retained in the host cell in such a manner that the gene can be expressed. These genes may be retained, for example, all on a single expression vector, or on a chromosome. These genes may also be retained, for example, separately on multiple expression vectors, or separately on a single or multiple expression vectors and on a chromosome. The genes exemplified above and the proteins encoded thereby may have, for example, nucleotide sequences and amino acid sequences of known genes and proteins, respectively. The genes exemplified above and proteins encoded thereby may also be, for example, conservative variants of known genes and proteins, respectively. For conservative variants of the genes and proteins, the descriptions concerning conservative variants of olfactory receptor genes and olfactory receptors can be applied.

A cell having an olfactory receptor gene may be used as it is, or may be used as a cell having an olfactory receptor (cell of the present invention) after the olfactory receptor gene is appropriately expressed. In other words, if a cell having an olfactory receptor gene already expresses the olfactory receptor gene, the cell may be used as it is as a cell having an olfactory receptor (cell of the present invention). By allowing a cell having an olfactory receptor gene to express the olfactory receptor gene, a cell having an olfactory receptor (cell of the present invention) can also be obtained. For example, by culturing a cell having an olfactory receptor gene, the olfactory receptor gene can be expressed, and thereby a cell having an olfactory receptor (cell of the present invention) can be obtained. Specifically, for example, after introduction (e.g. transfection) of an olfactory receptor gene, culture of the host cell can be continued to allow expression of the olfactory receptor gene. The medium composition and culture conditions are not particularly limited so long as the cell having the olfactory receptor gene can be maintained (e.g., proliferated) and the olfactory receptor gene is expressed. During the culture, cells having the olfactory receptor gene may or may not proliferate. The medium composition and culture conditions can be appropriately determined according to various conditions, such as type of the host cell. For example, the culture can be performed by using a usual culture medium and usual conditions used for culturing cells such as animal cells as they are, or with appropriate modifications. For example, specific examples of media that can be used for culturing animal cells include Opti-MEM medium (Thermo Fisher Scientific), DMEM medium, RPMI 1640 medium, and CD293 medium. The culture can be performed, for example, as static culture at 36 to 38°C in an atmosphere containing $CO_2$ such as 5% $CO_2$. A selection agent and expression inducer can be used if necessary.

Expression of the olfactory receptor can be confirmed by measuring a response of the olfactory receptor to the olfactory receptor-activating substance (for example, activation of the olfactory receptor by the olfactory receptor-activating substance). Expression of the olfactory receptor can also be confirmed by measuring amount of mRNA transcribed from the olfactory receptor gene or by detecting the olfactory receptor by Western blotting using antibodies.

The cell of the present invention may be used in the screening method of the present invention, for example, either as it is (as contained in the culture medium) or after collected from the medium. The culture medium or the cells recovered therefrom may also be used in the screening method of the present invention after such a process as, for example, washing, concentration, dilution, and immobilization, as appropriate. As described above, for example, the cell of the present invention may be used in the form of cells isolated to a desired degree, or may be used in the form of cells contained in any material such as culture product. The same shall apply to other structures having the olfactory receptor.

A cell membrane having the olfactory receptor can be prepared from, for example, the cell of the present invention. Specifically, cell membranes having the olfactory receptor can be obtained, for example, as a membrane fraction when the cells of the present invention are disrupted. Cell membranes having the olfactory receptor may be used, for example, as they are or after dispersed in an artificial lipid bilayer membrane. Cell membranes having the olfactory receptor may also be used in the form of vesicles (i.e., vesicles prepared from the cell membranes).

The olfactory receptor can also be used to produce artificial lipid bilayer membrane vesicles or artificial lipid bilayer membranes having the olfactory receptor. For example, artificial lipid bilayer membrane vesicles or artificial lipid bilayer membranes having the olfactory receptor can be prepared by incorporating the olfactory receptor into artificial lipid bilayer membrane vesicles or artificial lipid bilayer membranes prepared beforehand. Artificial lipid bilayer membrane vesicles or artificial lipid bilayer membranes having the olfactory receptor can also be prepared by, for example, preparing the olfactory receptor in artificial lipid bilayer membrane vesicles or artificial lipid bilayer membranes using the olfactory receptor as a raw material. For the preparation of artificial lipid bilayer membrane vesicles or artificial lipid bilayer membranes having the olfactory receptor, the olfactory receptor in an appropriate form such as a fraction of membranes having the olfactory receptor can be used. Such artificial lipid bilayer membrane vesicles and artificial lipid bilayer membranes can be produced by, for example, known means. Examples of the method for producing artificial lipid bilayer membranes include, for example, the Montal-Mueller method, and the droplet contact method (Kawano R. et al., Automated Parallel Recordings of Topologically Identified Single Ion Channels, Scientific Reports, 3, No. 1995 (2013)). For example, US 2018/0095071 A1 discloses preparation of artificial lipid bilayer membranes using a crude purified membrane fraction obtained from cultured cells. The artificial lipid bilayer membrane vesicles may have the olfactory receptor, e.g., in the membranes thereof. Examples of the lipid bilayer membrane vesicles include liposomes.

Membranes such as cell membranes and artificial lipid bilayer membranes can be used, for example, to create spaces that are demarcated by such membranes. Such membranes can be used, for example, to demarcate two spaces, such as two wells. That is, such membranes can be used, for example, to provide a reaction system with two spaces, such as two wells, wherein the two spaces are demarcated from each other by the membranes. Such two spaces need only be demarcated by the cell membranes for at least a part of the boundary thereof. Such a reaction system may be provided, for example, as the device described above.

<1-5> Screening method of the present invention

The screening method of the present invention can be performed in vitro.

The step (A) is a step of contacting an olfactory receptor with an olfactory receptor-activating substance in the presence of a test substance. That is, the olfactory receptor can be first contacted with the olfactory receptor-activating substance in the presence of the test substance. In other words, the olfactory receptor can be contacted with the test substance in the presence of the olfactory receptor-activating substance. Also in other words, the olfactory receptor can be contacted with the olfactory receptor-activating substance and the test substance. That is, the expressions "contacting an olfactory receptor with an olfactory receptor-activating substance in the presence of a test substance," "contacting an olfactory receptor with a test substance in the presence of an olfactory receptor-activating substance," and "contacting an olfactory receptor with an olfactory receptor-activating substance and a test substance" can be used synonymously. Hereafter, the olfactory receptor-activating substance and test substance are also collectively referred to as "both substances".

The system in which the olfactory receptor and both substances are contacted is also referred to as the "reaction system".

The contact between the olfactory receptor and both substances can be carried out in an appropriate liquid. The liquid in which the contact between the olfactory receptor and both substances is carried out is also referred to as the "reaction liquid". That is, examples of the reaction system include the reaction liquid. For example, the olfactory receptor and both substances can be contacted by allowing them to coexist in an appropriate reaction liquid. Specifically, for example, the olfactory receptor (e.g., in any of the forms exemplified above such as cells having an olfactory receptor) and both substances can be contacted with each other by dissolving, suspending, or dispersing them in an appropriate liquid medium, or similar methods. Examples of the liquid medium include aqueous media such as water and aqueous buffer. When two or more types of ingredients such as the both substances are together contacted with the olfactory receptor, the contacts between the ingredients and the olfactory receptor may or may not be started simultaneously. That is, for example, after starting contact of one ingredient with the olfactory receptor, another ingredient may be further added to the reaction system. Specifically, for example, an olfactory receptor-activating substance may be further added to the reaction system after starting contact between the test substance and the olfactory receptor, or the test substance may be further added to the reaction system after starting contact between the olfactory receptor-activating substance and the olfactory receptor. Normally, the test substance and the olfactory receptor-activating substance can be mixed beforehand, and contacted with the olfactory receptor. The reaction conditions (conditions under which contact between the olfactory receptor and both substances is carried out) are not particularly limited as long as it is possible to screen for substances that suppresses 4-methylacetophenone odor. The reaction conditions can be appropriately set according to various conditions, such as the form of the olfactory receptor used, type of test substance, and method used to measure a response of the olfactory receptor. For example, known reaction conditions for measuring interactions between substances, such as interactions between proteins and ligands, may be used as they are or with any appropriate modifications. The concentration of the test substance may be, for example, 0.01 nM to 500 mM, 10 nM to 100 mM, 1 $\mu$M to 10 mM, or 3 $\mu$M to 1 mM. The concentration of the olfactory receptor-activating substance may be, for example, 0.01 nM to 500 mM, 10 nM to 100 mM, 1 $\mu$M to 10 mM, or 3 $\mu$M to 1 mM. The concentration of the olfactory receptor may be, for example, 1 pg/mL to 10 mg/mL. When cells having the olfactory receptor are used, the density of the cells having the olfactory receptor may be, for example, 10 to 10,000,000 cells/mL. The contact between the olfactory receptor and both substances may or may not be terminated at an appropriate time. The contact between the olfactory receptor and both substances may normally be continued until the time of measurement of the response of the olfactory receptor to the olfactory receptor-activating substance. The duration of the contact between the olfactory receptor and both substances may be, for example, 0.1 second or longer, 0.5 second or longer, 1 second or longer, 5 seconds or longer, 10 seconds or longer, 30 seconds or longer, 1 minute or longer, 5 minutes or longer, 10 minutes or longer, 30 minutes or longer, 1 hour or longer, or 2 hours or longer, and 24 hours or shorter, 12 hours or shorter, 6 hours or shorter, 2 hours or shorter, or 1 hour or shorter, or in any range defined by any non-contradictory combination of these exemplary minimum and maximum durations. The duration of the contact between the olfactory receptor and both substances may specifically be, for example, 1 to 6 hours. The reaction system may contain another ingredient in addition to the olfactory receptor (in any of the forms exemplified above such as cells having the olfactory receptor) and both substances, as long as the screening for substances that suppress 4-methylacetophenone odor is possible. The other ingredient can be chosen according to various conditions, such as the form of olfactory receptor used, type of the test substance, and method used to measure a response of the olfactory receptor. Examples of the other ingredient include salts such as calcium salts, carbon sources such as glucose, other medium ingredients, and pH-buffering agents.

The step (B) is a step of measuring a response of the olfactory receptor to the olfactory receptor-activating substance. That is, a response of the olfactory receptor to the olfactory receptor-activating substance can then be measured. The fact that the olfactory receptor responds to the olfactory receptor-activating substance is also expressed as "the olfactory receptor-activating substance elicits a response of the olfactory receptor". The response of the olfactory receptor to an olfactory receptor-activating substance serves as an indicator for evaluating inhibition of the response by the test substance, as described later. Therefore, the expression "measuring a response of the olfactory receptor to the olfactory receptor-activating substance" may specifically mean measuring inhibition of the response by the test substance. The term "inhibition of the response by the test substance" means that the response of the olfactory receptor to the olfactory receptor-activating substance is inhibited by the test substance.

Examples of the response of the olfactory receptor to the olfactory receptor-activating substance include activation of the olfactory receptor by the olfactory receptor-activating substance.

The timing for measuring the response of the olfactory receptor to the olfactory receptor-activating substance is not particularly limited as long as when inhibition of the response by the test substance has occurred to a measurable degree if the test substance is a substance that suppresses 4-methylacetophenone odor. The timing for measuring the response of the olfactory receptor to the test substance can be set according to various conditions, such as the form of the olfactory receptor used, types of both substances, and method used to measure a response of the olfactory receptor. Specifically, the timing for measuring a response of the olfactory receptor to the olfactory receptor-activating substance may be an appropriate time point from the time when the contact between the olfactory receptor and both substances is started to the time when inhibition of the response by the test substance disappears. The timing for measuring a response of the olfactory receptor to the olfactory receptor-activating substance may be, for example, a time point when the inhibition of the response by the test substance is maximized. The timing for measuring a response of the olfactory receptor to the olfactory receptor-activating substance may be, for example, a time point after a period of 0.1 second or longer, 0.5 second or longer, 1 second or longer, 5 seconds or longer, 10 seconds or longer, 30 seconds or longer, 1 minute or longer, 5 minutes or longer, 10 minutes or longer, 30 minutes or longer, 1 hour or longer, or 2 hours or longer, and within 24 hours, 12 hours, 6 hours, 2 hours, or 1 hour from the start of the contact between the olfactory receptor and both substances, or in any range defined by a non-contradictory combination of these earliest and latest time points. The timing for measuring a response of the olfactory receptor to the olfactory receptor-activating substance may be specifically, for example, within a range of from 1 hour to 6 hours after the time point when the contact between the olfactory receptor and both substances has been started.

The step (C) is a step of identifying the test substance as a substance that suppresses 4-methylacetophenone odor on the basis of the response of the olfactory receptor to the olfactory receptor-activating substance. That is, it is then possible to identify whether the test substance is a substance that suppresses 4-methylacetophenone odor on the basis of the response of the olfactory receptor to the olfactory receptor-activating substance. In other words, the test substance can be identified as a substance that suppresses 4-methylacetophenone odor on the basis of the response of the olfactory receptor to the olfactory receptor-activating substance.

Specifically, inhibition of the response by the test substance can be evaluated on the basis of the response of the olfactory receptor to the olfactory receptor-activating substance, and furthermore, the test substance can be identified as a substance that suppresses 4-methylacetophenone odor on the basis of inhibition of the response by the test substance. More specifically, when inhibition of the response by the test substance is observed, i.e., when the response of the olfactory receptor to the olfactory receptor-activating substance is inhibited by the test substance, the test substance can be identified as a substance that suppresses 4-methylacetophenone odor. In other words, for example, if activation of the olfactory receptor by the olfactory receptor-activating substance is inhibited by the test substance, the test substance can be identified as a substance that suppresses 4-methylacetophenone odor. Inhibition of the activation of the olfactory receptor by the olfactory receptor-activating substance by a test substance is also referred to as "inhibition of activation by a test substance" or "inactivation of olfactory receptor by a test substance".

The inactivation of the olfactory receptor by a test substance can be determined by using the degree of activation of the olfactory receptor (degree of activation D1) observed when the step (A) mentioned above is performed (i.e., under the condition where the olfactory receptor and the olfactory receptor-activating substance are contacted in the presence of the test substance) as an indicator. In other words, the step (B) mentioned above can be, for example, a step (B1) of measuring the degree of activation D1. The aforementioned step (C) may be, for example, a step (C1) of identifying whether the test substance is a substance that suppresses 4-methylacetophenone odor on the basis of the degree of activation D1.

The inactivation of the olfactory receptor by the test substance can be determined specifically by comparing the degree of activation of the olfactory receptor (degree of activation D1) observed when the above step (A) is performed (i.e., under the condition where the olfactory receptor and the olfactory receptor-activating substance are contacted in the presence of the test substance) with degree of activation of the olfactory receptor observed under a control condition (degree of activation D2). In other words, the aforementioned step (C1) may be, for example, a step (C2) of identifying whether the test substance is a substance that suppresses 4-methylacetophenone odor on the basis of the difference between the degree of activation D1 and the degree of activation D2.

The "control condition" means the following condition (C2-1) or (C2-2):

(C2-1) a condition under which the olfactory receptor is contacted with the olfactory receptor-activating substance in the absence of the test substance; or
(C2-2) a condition under which the olfactory receptor is contacted with the olfactory receptor-activating substance in the presence of the test substance, and the concentration of the test substance is lower than the concentration of the test substance used in the step (A) mentioned above.

In other words, the inactivation of the olfactory receptor by the test substance can be determined by, for example, using the difference in the degree of activation of the olfactory receptor caused by difference in the presence and absence or difference in concentration of the test substance in the presence of the olfactory receptor-activating substance as an indicator.

Examples of the aforementioned condition (C2-1) include a condition where the olfactory receptor is contacted with the olfactory receptor-activating substance before the olfactory receptor is contacted with the test substance. Examples of the aforementioned condition (C2-1) also includes a condition where the test substance is substantially (e.g., completely) removed from the reaction system and the inhibition of the response by the test substance is substantially (e.g., completely) eliminated after the olfactory receptor is contacted with the olfactory receptor-activating substance and the test substance. The concentration of the test substance of the condition (C2-2) is not particularly limited as long as it is such a concentration that a measurable difference is observed between the degree of activation D1 and the degree of activation D2. The concentration of the test substance of the condition (C2-2) may be, for example, 90% or less, 70% or less, 50% or less, 30% or less, 20% or less, 10% or less, 5% or less, or 1% or less of the concentration of the test substance used in the above step (A). The control condition is not particularly limited except for the difference in the presence or absence or in concentration of the test substance, as long as inhibition of the response by the test substance can be evaluated. The control condition except for the difference in the presence or absence or in concentration of the test substance may be, for example, the same as the condition of the above step (A).

The screening method of the present invention may comprise the step of measuring the degree of activation D2. The degree of activation D1 and the degree of activation D2 may be measured in a single reaction system with a time lag, or they may be measured simultaneously or with a time lag in separate reaction systems. The degree of activation D2 may be measured before or after measuring the degree of activation D1. For example, after measuring the degree of activation D2, the test substance may be added to the reaction system, and the degree of activation D1 may be measured.

When the degree of activation D1 is low, it may be determined that inactivation of the olfactory receptor by the test substance has been observed. Specifically, when the degree of activation D1 is lower than the degree of activation D2, it may be determined that inactivation of the olfactory receptor by the test substance has been observed. For example, if the ratio of the degree of activation D1 to the degree of activation D2 is lower than 80%, lower than 70%, lower than 60%, lower than 50%, lower than 40%, lower than 30%, lower than 20%, or lower than 10%, it may be determined that inactivation of the olfactory receptor by the test substance has been observed. Such ratio is also referred to as the "residual ratio of activity". The value obtained by subtracting the residual ratio of activity from 100% is also referred to as "inhibition ratio of activity". Specific examples of the inhibition ratio of activity include, for example, the inhibition ratios described in the section of Examples.

The above description for determining inactivation of the olfactory receptor by the test substance can be applied to determining inhibition of the response by the test substance on the basis of another indicator. In such a case, the term "degree of activation" in the above description can be replaced with a term corresponding to the other indicator as appropriate.

The method for measuring the response of the olfactory receptor to the olfactory receptor-activating substance is not particularly limited. The method for measuring the response of the olfactory receptor to the olfactory receptor-activating substance can be selected according to various conditions, such as the form of the olfactory receptor used and type of the response to be measured. In other words, the response of the olfactory receptor to the olfactory receptor-activating substance can be measured by, for example, an appropriate technique that enables measurement of activation of the olfactory receptor by the olfactory receptor-activating substance.

The method for measuring activation of the olfactory receptor by the olfactory receptor-activating substance is not particularly limited. Activation of the olfactory receptor by the olfactory receptor-activating substance can be measured by, for example, known methods for measuring activity of receptors such as olfactory receptors. Examples of such methods include, for example, methods for measuring intracellular calcium concentration and methods for measuring intracellular cAMP concentration. In other words, activation of the olfactory receptor by the olfactory receptor-activating substance can be measured by, for example, using intracellular calcium concentration or intracellular cAMP concentration. That is, activation of the olfactory receptor by the olfactory receptor-activating substance can be measured specifically by, for example, using cells having the olfactory receptor and intracellular calcium concentration or intracellular cAMP concentration as an indicator. For example, it is known that in HEK293T cells, when olfactory receptors are activated by odorants, they conjugate with intracellular G proteins (e.g., Golf) to activate adenylate cyclase, thereby increasing the amount of intracellular cAMP (Kajiya K. et al., Molecular bases of odor discrimination: Reconstitution of olfactory receptors that recognize overlapping sets of odorants. Journal of Neuroscience, 2001, 21: 6018-6025). Examples of the methods for measuring intracellular cAMP levels include, for example, ELISA and reporter assay. Examples of the reporter assay include luciferase assay. By the reporter assay, intracellular cAMP concentration can be measured by using a reporter gene (such as luciferase gene) that is constituted to be expressed in a cAMP concentration-dependent manner. Examples of the methods for measuring intracellular calcium concentration include, for example, calcium imaging. The calcium imaging enables measurement of intracellular calcium concentration by using a calcium indicator. Examples of the calcium indicator include calcium-sensitive fluorescent dyes and calcium-sensitive fluorescent proteins. Examples of the calcium-sensitive fluorescent dyes include Fura 2 and Fluo 4. Examples of the calcium-sensitive fluorescent proteins include, for example, Cameleon, TN-XL, GCaMP, and G-GECO. The term "calcium concentration" may mean concentration of free calcium ions.

The description for the measurement of activation of the olfactory receptor using cells having the olfactory receptor can also be applied to the case of using the olfactory receptor in other forms. Examples of the case of using the olfactory receptor in other forms include the case of using artificial lipid bilayer membrane vesicles, cell membranes, or artificial lipid bilayer membranes having the olfactory receptor. Examples of the case of using the olfactory receptor in other forms include, especially, use of the olfactory receptor in such a form that the olfactory receptor has an internal space.

That is, activation of the olfactory receptor can be measured by, for example, using artificial lipid bilayer membrane vesicles having the olfactory receptor in the same manner as the case of using cells having the olfactory receptor. In such a case, the term "cell" in the description for the measurement of activation of the olfactory receptor using cells having the olfactory receptor can be read as "artificial lipid bilayer membrane vesicle".

Further, activation of the olfactory receptor can be measured by, for example, using membranes such as cell membranes or artificial lipid bilayer membranes having the olfactory receptor so that spaces demarcated by the membranes are created in the same manner as the cases of using cells having the olfactory receptor. Specifically, for example, when such membranes are used so that each membrane demarcates two spaces, i.e., when each membrane provides a reaction system having two spaces, wherein the two spaces are demarcated from each other by the membrane, activation of the olfactory receptor can be measured in a manner similar to that used in the case of using cells having the olfactory receptor. In such a case, the space demarcated by the membrane can be considered the inside of the cell (also referred to as the "internal space"). Specifically, one of the two spaces can be regarded as the inside of the cell (also called "internal space") and the other as the outside of the cell (also called "external space"). Of those spaces, the one containing the both substances can be considered the external space. In such a case, the terms "intracellular calcium concentration" and "intracellular cAMP concentration" in the description for the measurement of activation of the olfactory receptor using cells having the olfactory receptor can be read as "calcium concentration in the internal space" and "cAMP concentration in the internal space", respectively.

In any case, measurable parameters can be selected according to the mode of using the olfactory receptor.

For "measuring a certain parameter and using it as an indicator for measuring a response of the olfactory receptor to the olfactory receptor-activating substance", it is sufficient to obtain and use data reflecting the parameter, and it is not necessary to obtain the value of the parameter itself, as long as the response can be measured, specifically, as long as it is possible to evaluate inhibition of the response by the test substance on the basis of the response (i.e., it is possible to determine whether inhibition of the response by the test substance is recognized). In other words, when data reflecting a parameter are obtained, it is not necessary to calculate the value of the parameter itself from the data. Specifically, for example, when the intracellular cAMP concentration is measured by a luciferase assay and used as an indicator to measure activation of the olfactory receptor by the olfactory receptor-activating substance, it is sufficient to obtain and use data reflecting the intracellular cAMP concentration (e.g., emission intensity) and it is not necessary to calculate the intracellular cAMP concentration itself from the data, as long as it is possible to evaluate inactivation of the olfactory receptor by the test substance on the basis of such activation (that is, as long as it is possible to determine whether inactivation of the olfactory receptor by the test substance is recognized).

In addition, for "measuring a response of the olfactory receptor to the olfactory receptor-activating substance", it is sufficient to obtain data that reflect such a response and can be used to evaluate inhibition of the response by the test substance. Similarly, it is sufficient that the "response of the olfactory receptor to the olfactory receptor-activating substance" used as an indicator for evaluating inhibition of the response by the test substance is data reflecting such a response and can be used to evaluate inhibition of the response by the test substance. As such data, for example, data obtained by implementing a method for measuring a response of the olfactory receptor to the olfactory receptor-activating substance (e.g., parameters exemplified above and data reflecting them) can be used as they are or after appropriate processing.

As described above, a substance that suppresses 4-methylacetophenone odor can be identified. The screening method of the present invention may further comprise the step of evaluating a masking function of the identified substance that suppresses 4-methylacetophenone odor (i.e., evaluating whether the identified substance that suppresses 4-methylacetophenone odor has a masking function). That is, by evaluating masking function of the identified substance that suppresses 4-methylacetophenone odor, whether the substance that suppresses 4-methylacetophenone odor actually suppresses 4-methylacetophenone odor can be confirmed. The method for evaluating masking function of the identified substance that suppresses 4-methylacetophenone odor is not particularly limited. Masking function of the identified substance that suppresses 4-methylacetophenone odor can be evaluated by, for example, known methods for evaluating aromas of substances. Examples of such methods include sensory evaluation (evaluation by sensory test). Masking function of the identified substance that suppresses 4-methylacetophenone odor can be specifically evaluated by, for example, comparing 4-methylacetophenone odors of an object that presents 4-methylacetophenone odor (e.g., a food containing 4-methylacetophenone) felt in the presence and absence of the substance that suppresses 4-methylacetophenone odor.

In the conventional screening methods, in order to screen for 4-methylacetophenone odor-suppressing agents, a vast number of substances or combinations of substances must be tested one by one for confirming masking function thereof by sensory test etc. to select substances that suppress 4-methylacetophenone odor. Therefore, they require a lot of time and costs to develop a substance that suppresses 4-methylacetophenone odor. However, according to the screening method of the present invention, it is possible to efficiently screen substances for a substance that suppresses 4-methylacetophenone odor by using an olfactory receptor. Therefore, the screening method of the present invention can greatly improve the efficiency of the development of 4-methylacetophenone odor-suppressing agent.

Use of the substance that suppresses 4-methylacetophenone odor selected by the screening method is not particularly limited. A substance that suppresses 4-methylacetophenone odor can be used by, for example, formulating it in an object for which suppression of 4-methylacetophenone odor is desired. Examples of such an object include those that already present 4-methylacetophenone odor (e.g., foods containing 4-methylacetophenone) and those that may present 4-methylacetophenone odor in the future (e.g., foods containing an ingredient that may generate 4-methylacetophenone such as citral). 4-Methylacetophenone odor of an object can be suppressed by formulating a substance that suppresses 4-methylacetophenone odor. For the suppression of 4-methylacetophenone odor using a substance that suppresses 4-methylacetophenone odor, for example, the description for the suppression of 4-methylacetophenone odor using an active ingredient in the section "<2> Suppression of 4-methylacetophenone odor" mentioned below. Further, a substance that suppresses 4-methylacetophenone odor can also be used as, for example, a raw material for the development of novel substances that suppress 4-methylacetophenone odor.

<2> Suppression of 4-methylacetophenone odor

<2-1> Active ingredient

For the suppression of 4-methylacetophenone odor, the following ingredient (A) is used as an active ingredient:

(A) at least one type of ingredient selected from the group consisting of N-vanillylnonanamide, nootkatone, 2-phenylethanethiol, benzyl isothiocyanate, 2-thiophenemethanethiol, difurfuryl sulfide, 2-methylbenzenethiol, benzyl isoeugenol, 2-phenyl-2-butenal, 4-methyl-1-phenyl-2-pentanol, 2-thiophenethiol, 2,4-heptadienal, and S-(2-methyl-3-furyl) ethanethioate.

The ingredient (A) is also referred to as the "active ingredient". As the ingredient (A), one type of ingredient may be used, or a combination of two or more types of ingredients may be used. The combination for the active ingredient is not particularly limited.

All of ingredients as the active ingredient may be an ingredient that inactivates the olfactory receptor OR5P3. In other words, the active ingredient may specifically be the following ingredient (A):

(A) at least one type of ingredient that inactivates the olfactory receptor OR5P3 and is selected from the group consisting of N-vanillylnonanamide, nootkatone, 2-phenylethanethiol, benzyl isothiocyanate, 2-thiophenemethanethiol, difurfuryl sulfide, 2-methylbenzenethiol, benzyl isoeugenol, 2-phenyl-2-butenal, 4-methyl-1-phenyl-2-pentanol, 2-thiophenethiol, 2,4-heptadienal, and S-(2-methyl-3-furyl) ethanethioate.

The expression that "a certain ingredient inactivates the olfactory receptor OR5P3" means that inactivation of the olfactory receptor OR5P3 by the ingredient is recognized under appropriate conditions. The olfactory receptor OR5P3 and inactivation thereof are as described above in the section "<1> Screening for substance that suppresses 4-methylacetophenone odor". Examples of the appropriate conditions include those described above in the section "<1> Screening for substance that suppresses 4-methylacetophenone odor". Specific examples of the appropriate conditions include the conditions described in the examples. That is, the expression that "a certain ingredient inactivates the olfactory receptor OR5P3" may mean that, for example, inactivation of the olfactory receptor OR5P3 by the ingredient is observed at least under the conditions described in the examples.

By utilizing the active ingredient, 4-methylacetophenone odor of foods can be suppressed, i.e., an effect of suppressing 4-methylacetophenone odor of foods can be obtained. This effect is also referred to as the "masking effect". Suppression of 4-methylacetophenone odor of foods is also simply referred to as "suppression of 4-methylacetophenone odor". The "suppression of 4-methylacetophenone odor" is also referred to as the "reduction of 4-methylacetophenone odor" or "masking of 4-methylacetophenone odor". The "suppression of 4-methylacetophenone odor" encompasses both suppression of 4-methylacetophenone odor that may be generated in the future and suppression of 4-methylacetophenone odor that has already been generated. The "suppression of 4-methylacetophenone odor" also encompasses making 4-methylacetophenone odor completely disappear. The term "4-methylacetophenone odor" refers to odor presented by 4-methylacetophenone. 4-Methylacetophenone can be a causative substance of citral-derived degradation odor. Therefore, suppression of 4-methylacetophenone odor may suppress, for example, citral-derived degradation odor. In addition, suppression of 4-methylacetophenone odor may suppress, for example, degradation odor of an object (e.g., food) containing citral. Citral may be contained in citrus fruits such as lemon and herbs such as lemongrass. Specifically, by suppressing 4-methylacetophenone odor, for example, degradation odor derived from lemon (e.g. lemon juice) may be suppressed. Specifically, use of the active ingredient may suppress 4-methylacetophenone odor of a food compared with the case where the active ingredient is not used. Therefore, the masking effect can be determined by measuring and comparing 4-methylacetophenone odors in foods utilizing and not utilizing the active ingredient. That is, if strength of 4-methylacetophenone odor of a food utilizing the active ingredient is lower than that of a food not utilizing the active ingredient, it can be determined that the masking effect has been obtained. Degradation odors such as 4-methylacetophenone odor can be measured and compared by, for example, sensory evaluation performed by a panel of experts.

Degradation odors such as 4-methylacetophenone odor may be classified into, for example, initial, middle, and after degradation odors. In the case of liquids (in the case of liquid foods), the terms "initial", "middle", and "after" used for degradation odors such as 4-methylacetophenone odor are used to mean degradation odors sensed in the periods of 0 to 1 second, 1 to 3 seconds, and 3 to 5 seconds after eating (after the food is put into the mouth), respectively. In the case of solids (in the case of solid foods), the terms "initial", "middle", and "after" used for degradation odors such as 4-methylacetophenone odor are used to mean degradation odors sensed in the periods of 0 to 4 seconds, 4 to 10 seconds, and 10 to 15 seconds after eating (after the food is put into the mouth), respectively. For the present invention, the term "solid" means a form other than liquid, and encompasses paste, gel, and so forth. By utilizing the active ingredient, for example, initial degradation odor, middle degradation odor, after degradation odor, or a combination thereof may be suppressed. By utilizing the active ingredient, in particular, initial and middle degradation odors or initial, middle and after degradation odors may be suppressed.

As the active ingredient, commercially available products may be used, or those appropriately produced and obtained may be used. The method for producing the active ingredient is not particularly limited. For example, the active ingredient can be produced by chemical synthesis, enzymatic reaction, fermentation, extraction, or a combination thereof. The active ingredient may or may not be purified to a desired degree. In other words, as the active ingredient, a purified product may be used, or a material containing the active ingredient may be used. For example, as the active ingredient, a material having a content of the active ingredient of 1% (w/w) or higher, 5% (w/w) or higher, 10% (w/w) or higher, 30% (w/w) or higher, 50% (w/w) or higher, 70% (w/w) or higher, 90% (w/w) or higher, or 95% (w/w) or higher may be used.

In the case of using a material containing the active ingredient, the amount of the active ingredient (e.g., content (concentration) or amount to be used) shall be calculated on the basis of the amount of the active ingredient itself in the material.

<2-2> Composition of the present invention

The composition of the present invention is a composition containing the active ingredient.

That is, the composition of the present invention is a composition containing the following ingredient (A):

(A) at least one type of ingredient selected from the group consisting of N-vanillylnonanamide, nootkatone, 2-phenylethanethiol, benzyl isothiocyanate, 2-thiophenemethanethiol, difurfuryl sulfide, 2-methylbenzenethiol, benzyl isoeugenol, 2-phenyl-2-butenal, 4-methyl-1-phenyl-2-pentanol, 2-thiophenethiol, 2,4-heptadienal, and S-(2-methyl-3-furyl) ethanethioate.

The composition of the present invention may specifically be a composition containing the following ingredient (A):

(A) at least one type of ingredient that inactivates the olfactory receptor OR5P3 and is selected from the group consisting of N-vanillylnonanamide, nootkatone, 2-phenylethanethiol, benzyl isothiocyanate, 2-thiophenemethanethiol, difurfuryl sulfide, 2-methylbenzenethiol, benzyl isoeugenol, 2-phenyl-2-butenal, 4-methyl-1-phenyl-2-pentanol, 2-thiophenethiol, 2,4-heptadienal, and S-(2-methyl-3-furyl) ethanethioate.

By utilizing the composition of the present invention, 4-methylacetophenone odor of a food can be suppressed, i.e., the masking effect can be obtained. Therefore, the composition of the present invention may be utilized for suppressing 4-methylacetophenone odor of a food. That is, the composition of the present invention may be, for example, a composition for suppressing 4-methylacetophenone odor of a food.

By utilizing the composition of the present invention, a food of which 4-methylacetophenone odor is suppressed can also be produced. Therefore, the composition of the present invention may be utilized in production of a food (specifically, production of a food of which 4-methylacetophenone odor is suppressed). That is, the composition of the present invention may be, for example, a composition for producing a food (specifically, production of a food of which 4-methylacetophenone odor is suppressed).

The composition of the present invention may be, for example, a seasoning. The composition of the present invention may specifically be, for example, a seasoning for suppressing 4-methylacetophenone odor of a food, and may be a seasoning for producing a food (specifically, production of a food of which 4-methylacetophenone odor is suppressed).

The composition of the present invention may be utilized for suppressing 4-methylacetophenone odor of a food or for producing a food in the manner described for the second embodiment of the method of the present invention described later.

The composition of the present invention may consist of the active ingredient or may contain an ingredient other than the active ingredient. The composition consisting of the active ingredient may be excluded from the composition of the present invention. As the ingredient other than the active ingredient, one type of ingredient may be used, or a combination of two or more types of ingredients may be used.

The ingredient other than the active ingredient is not particularly limited as long as the masking effect is not impaired. The ingredient other than the active ingredient can be selected according to various conditions, such as, for example, the type of food. Examples of the ingredient other than the active ingredient include ingredients used in foods or pharmaceuticals. Specific examples of the ingredient other than the active ingredient include the raw materials of foods described below. Specific examples of the ingredient other than the active ingredient also include sclareol. Sclareol may be used in combination with any type of the active ingredient. Sclareol may be used in combination with, for example, nootkatone and/or S-(2-methyl-3-furyl) ethanethioate. The combination of sclareol with the active ingredient may, for example, improve the masking effect compared with the use of the active ingredient alone.

The composition of the present invention can be produced by, for example, mixing the active ingredient and optionally another ingredient as appropriate.

The composition of the present invention may be, for example, appropriately made into a preparation. When the composition of the present invention is made into a preparation, an additive may be used as appropriate. Examples of the additive include excipient, binder, disintegrating agent, lubricant, stabilizer, flavoring agent, diluent, surfactant, and solvent. The additive can be appropriately selected depending on various conditions, such as the form of the composition of the present invention.

The form of the composition of the present invention is not particularly limited. The composition of the present invention may be in an arbitrary form, for example, in the form of powder, flake, tablet, paste, liquid, or the like.

The contents and content ratios of the ingredients (i.e., active ingredient and optionally other ingredient) in the composition of the present invention are not particularly limited as long as the masking effect can be obtained. The contents and content ratios of the ingredients in the composition of the present invention can be appropriately set according to various conditions, such as the mode of use of the composition of the present invention.

The content of the active ingredient in the composition of the present invention is more than 0% (w/w) and not more than 100% (w/w). The content of the active ingredient in the composition of the present invention is, for example, 1 ppt (w/w) or higher, 10 ppt (w/w) or higher, 100 ppt (w/w) or higher, 1 ppb (w/w) or higher, 10 ppb (w/w) or higher, 100 ppb (w/w) or higher, 1 ppm (w/w) or higher, 10 ppm (w/w) or higher, 100 ppm (w/w) or higher, 1000 ppm (w/w) or higher, 1% (w/w) or higher, 2% (w/w) or higher, 5% (w/w) or higher, or 10% (w/w) or higher, and may be 100% (w/w) or lower, lower than 100% (w/w), 99.9% (w/w) or lower, 90% (w/w) or lower, 50% (w/w) or lower, 20% (w/w) or lower, 10% (w/w) or lower, 5% (w/w) or lower, 2% (w/w) or lower, 1% (w/w) or lower, 1000 ppm (w/w) or lower, 100 ppm (w/w) or lower, 10 ppm (w/w) or lower, or 1 ppm (w/w) or lower, or may be in a range defined by any non-contradictory combination of these exemplary minimum and maximum contents. The content of the active ingredient in the composition of the present invention may specifically be, for example, 1 ppt (w/w) to 1 ppm (w/w), 1 ppm (w/w) to 10 ppm (w/w), 10 ppm (w/w) to 100 ppm (w/w), 100 ppm (w/w) to 1000 ppm (w/w), 1000 ppm (w/w) to 1% (w/w), 1% (w/w) to 10% (w/w), or 10% (w/w) to 20% (w/w). The content of the active ingredient in the composition of the present invention may specifically be, for example, 1 ppt (w/w) to 10% (w/w), 1 ppt (w/w) to 1% (w/w), or 1 ppt (w/w) to 1000 ppm (w/w).

The contents of each ingredient (i.e., the active ingredient and optionally the other ingredient) in the composition of the present invention can be set, for example, to obtain the addition amounts of each ingredient used in the second embodiment of the method of the present invention described below.

Each ingredient (i.e., the active ingredient and optionally the other ingredient) contained in the composition of the present invention may be contained in the composition of the present invention as a mixture thereof, separately from each other, or separately in any combination thereof. For example, the composition of the present invention may be provided as a set of individual ingredients, each packaged separately. In such a case, the ingredients included in the set can be used together as appropriate at the time of use.

<2-3> Second embodiment of the method of the present invention

The second embodiment of the method of the present invention is a method comprising the step of utilizing the active ingredient.

That is, the second embodiment of the method of the present invention is a method comprising the step of utilizing the following ingredient (A):

(A) at least one type of ingredient selected from the group consisting of N-vanillylnonanamide, nootkatone, 2-phenylethanethiol, benzyl isothiocyanate, 2-thiophenemethanethiol, difurfuryl sulfide, 2-methylbenzenethiol, benzyl isoeugenol, 2-phenyl-2-butenal, 4-methyl-1-phenyl-2-pentanol, 2-thiophenethiol, 2,4-heptadienal, and S-(2-methyl-3-furyl) ethanethioate.

The second embodiment of the method of the present invention may specifically be a method comprising the step of utilizing the following ingredient (A):

(A) at least one type of ingredient that inactivates the olfactory receptor OR5P3 and is selected from the group consisting of N-vanillylnonanamide, nootkatone, 2-phenylethanethiol, benzyl isothiocyanate, 2-thiophenemethanethiol, difurfuryl sulfide, 2-methylbenzenethiol, benzyl isoeugenol, 2-phenyl-2-butenal, 4-methyl-1-phenyl-2-pentanol, 2-thiophenethiol, 2,4-heptadienal, and S-(2-methyl-3-furyl) ethanethioate.

By the second embodiment of the method of the present invention, specifically by utilizing the active ingredient, 4-methylacetophenone odor of a food can be suppressed, i.e., the masking effect can be obtained. Therefore, the second embodiment of the method of the present invention may be implemented for suppressing 4-methylacetophenone odor of a food. That is, the second embodiment of the method of the present invention may be, for example, a method for suppressing 4-methylacetophenone odor of a food. This method is also referred to as the "masking method of the present invention".

Further, by the second embodiment of the method of the present invention, specifically by utilizing the active ingredient, a food of which 4-methylacetophenone odor is suppressed can be produced. Therefore, the second embodiment of the method of the present invention may be implemented for producing a food (specifically, producing a food of which 4-methylacetophenone odor is suppressed). That is, the second embodiment of the method of the present invention may be, for example, a method for producing a food (specifically, producing a food of which 4-methylacetophenone odor is suppressed). This method is also referred to as the "food production method of the present invention".

The active ingredient can be used to suppress 4-methylacetophenone odor or produce a food by adding it to a raw material of food during the production of the food. That is, examples of the utilization of the active ingredient include addition of the active ingredient to a raw material of food. That is, the second embodiment of the method of the present invention may specifically be, for example, a method for suppressing 4-methylacetophenone odor of a food, which comprises adding the active ingredient to a raw material of the food. The second embodiment of the method of the present invention may also specifically be, for example, a method for producing a food (specifically, producing a food of which 4-methylacetophenone odor is suppressed), which comprises adding the active ingredient to a raw material of the food. The "addition" is also referred to as "formulating ".

The active ingredient may be utilized in the second embodiment of the method of the present invention, for example, in the form of the composition of the present invention. In other words, examples of the "utilization of the active ingredient" include utilization of the composition of the present invention. For example, "addition of the active ingredient" encompasses addition of the composition of the present invention.

The food obtained by the second embodiment of the method of the present invention is also referred to as the "food of the present invention". The food of the present invention is specifically a food of which 4-methylacetophenone odor has been suppressed. In other words, the food of the present invention is a food to which the active ingredient has been added.

The suppression of 4-methylacetophenone odor or the production of the food may be conducted, for example, in the same manner as the production of ordinary foods, except for the utilization of the active ingredient. That is, the suppression of 4-methylacetophenone odor or the production of the food may be conducted by, for example, using the same raw materials and production conditions as in the production of ordinary foods, except that the active ingredient is utilized. In addition, any of the raw materials and production conditions of foods may be used with appropriate modifications for the suppression of 4-methylacetophenone odor or the production of the food.

The food is not particularly limited so long as it is a food for which suppression of 4-methylacetophenone odor is desired. The food may be a food already presenting 4-methylacetophenone odor or a food that may present 4-methylacetophenone odor in the future. Examples of the food already presenting 4-methylacetophenone odor include foods containing 4-methylacetophenone. Examples of the food that may present 4-methylacetophenone odor in the future include foods containing an ingredient that can generate 4-methylacetophenone. That is, the food may contain 4-methylacetophenone and/or an ingredient that can generate 4-methylacetophenone. 4-Methylacetophenone can be produced from, for example, citral. That is, examples of the ingredient that can generate 4-methylacetophenone include citral. Citral can decrease, for example, during heating or storage to generate 4-methylacetophenone. Citral contained in the food may be, for example, a purified product of citral or a material containing citral. Examples of the material containing citral include citrus fruits such as lemon and mandarin, and herbs such as lemongrass. The citrus fruits may be, for example, the whole fruit or part of a fruit. Examples of the part of a fruit include juice, pulp, peel, and essential oil. Examples of the citrus fruits include, in particular, lemon juice. The herbs may be, for example, a whole plant body or a part of plant body. Examples of the part of plant body include an essential oil. Examples of the food also include beverages. Examples of the food also include seasonings. The food may be, for example, a liquid or solid. Specific examples of the food include beverages such as soft drinks, alcoholic beverages, and soups; confectioneries such as jellies, ice cream, candies, cakes, tarts, mousses, bavarois, and gums; processed fruit products such as dried fruits; and seasonings such as dressings, sauces, lemon juice, ponzu (Japanese citrus fruit vinegar), and jams. The term "soft drinks" may mean non-alcoholic beverages (beverages having an alcohol concentration lower than 1%) excluding milk and dairy products. Specific examples of the soft drinks include water, fruit juices, vegetable juices, tea (black tea, etc.), coffee drinks (coffee, etc.), carbonated drinks, sports drinks, and jelly drinks. Specific examples of the soups include tom yam kung. Examples of the food include, in particular, the foods exemplified above, which contain 4-methylacetophenone and/or an ingredient that can generate 4-methylacetophenone. Examples of the food include, in more particular, the foods exemplified above, which have been produced by adding citral (e.g., purified citral or materials containing citral, such as lemon juice).

The form of the food at the time of being provided is not particularly limited. For example, the food may be provided in such a form that the food can be eaten (drunk) as it is, or in a form that requires preparation before or at the time of eating (drinking), such as concentrated or dried products. The food may also be provided in a form contained in any container, such as retort pouches, paper cartons, plastic bottles such as PET bottles, metal cans such as steel cans and aluminum cans, and glass bottles. The food is not limited to general foods, but may also be a so-called health food or medical food, such as nutritional supplements, functional foods, and foods for specified health uses. That is, for example, the foods exemplified above may be provided as general foods, or as health foods or medical foods.

The term "raw material of food" means a food material used to produce a food. The raw material of food is not particularly limited as long as it can be used to produce a food. The raw material of food can be selected according to various conditions, such as, for example, the type of food. Examples of the raw material of food include raw materials that can be normally used in the production of foods such as those exemplified above. Specific examples of the raw material of food include cereal products such as flour; seasoning ingredients such as sugars, inorganic salts, organic acids, nucleic acids, amino acids, and protein hydrolysates; dairy products such as milk, cheese, and butter; fruits; vegetables; eggs; spices; flavorings; oils and fats; alcohols; dietary fibers; and pH buffering agents.

The active ingredient may be added to the raw material of food at any stage of the production process of the food as long as the masking effect is obtained. In other words, the "raw material of food" to which the active ingredient is added may be at any stage of the food production process. For example, the term the "raw material of food" to which the active ingredient is added may encompass a finished food before the active ingredient is added. The active ingredient can be added to the raw material of food as it is or after it is prepared in a desired form, such as a solution, as appropriate. The term "addition of the active ingredient" can be used as a generic term to refer to any operation of making the active ingredient coexist with the raw material of food. The ingredient other than the active ingredient (e.g., 4-methylacetophenone or ingredient that can generate 4-methylacetophenone) may also be added to the raw material of food as appropriate. The description for the addition of the active ingredient may also be applied to the addition of the ingredient other than the active ingredient. Each ingredient (i.e., the active ingredient and optionally the other ingredient) may all be added to the raw material of food at the same time, or each may be added separately, or separately in any combination thereof, to the raw material of food. The order of the addition of the ingredients to the raw material of food is not particularly limited.

The addition amounts and addition amount ratios of each ingredient (i.e., the active ingredient and optionally the other ingredient) used in the second embodiment of the method of the present invention are not particularly limited as long as the masking effect is obtained. The addition amounts and addition amount ratios of each ingredient used in the second embodiment of the method of the present invention can be appropriately set according to various conditions, such as the type of the raw material of food and the type of the food.

The active ingredient may be added to the raw material of food, for example, so that the concentration of the active ingredient at the time of eating (drinking) is in a desired range (e.g., the range of the concentration of the active ingredient at the time of eating described below).

The concentration of the active ingredient at the time of eating may be, for example, 0.001 ppt (w/w) or higher, 0.002 ppt (w/w) or higher, 0.005 ppt (w/w) or higher, 0.01 ppt (w/w) or higher, 0.02 ppt (w/w) or higher, 0.05 ppt (w/w) or higher, 0.1 ppt (w/w) or higher, 0.2 ppt (w/w) or higher, 0.5 ppt (w/w) or higher, 1 ppt (w/w) or higher, 2 ppt (w/w) or higher, 5 ppt (w/w) or higher, 10 ppt (w/w) or higher, 20 ppt (w/w) or higher, 50 ppt (w/w) or higher, 100 ppt (w/w) or higher, 200 ppt (w/w) or higher, 500 ppt (w/w) or higher, 1 ppb (w/w) or higher, 2 ppb (w/w) or higher, 5 ppb (w/w) or higher, 10 ppb (w/w) or higher, 20 ppb (w/w) or higher, 50 ppb (w/w) or higher, 100 ppb (w/w) or higher, 200 ppb (w/w) or higher, 500 ppb (w/w) or higher, 1 ppm (w/w) or higher, 2 ppm (w/w) or higher, 5 ppm (w/w) or higher, 10 ppm (w/w) or higher, 20 ppm (w/w) or higher, or 50 ppm (w/w) or higher, and may be 100 ppm (w/w) or lower, 50 ppm (w/w) or lower, 20 ppm (w/w) or lower, 10 ppm (w/w) or lower, 5 ppm (w/w) or lower, 2 ppm (w/w) or lower, 1 ppm (w/w) or lower, 500 ppb (w/w) or lower, 200 ppb (w/w) or lower, 100 ppb (w/w) or lower, 50 ppb (w/w) or lower, 20 ppb (w/w) or lower, 10 ppb (w/w) or lower, 5 ppb (w/w) or lower, 2 ppb (w/w) or lower, 1 ppb (w/w) or lower, 500 ppt (w/w) or lower, 200 ppt (w/w) or lower, 100 ppt (w/w) or lower, 50 ppt (w/w) or lower, 20 ppt (w/w) or lower, 10 ppt (w/w) or lower, 5 ppt (w/w) or lower, 2 ppt (w/w) or lower, 1 ppt (w/w) or lower, 0.5 ppt (w/w) or lower, 0.2 ppt (w/w) or lower, 0.1 ppt (w/w) or lower, 0.05 ppt (w/w) or lower, 0.02 ppt (w/w) or lower, 0.01 ppt (w/w) or lower, 0.005 ppt (w/w) or lower, or 0.002 ppt (w/w) or lower, or may be in a range defined by any non-contradictory combination of these exemplary minimum and maximum concentrations. The concentration of the active ingredient at the time of eating may specifically be, for example, 0.001 ppt (w/w) to 0.002 ppt (w/w), 0.002 ppt (w/w) to 0.005 ppt (w/w), 0.005 ppt (w/w) to 0.01 ppt (w/w), 0.01 ppt (w/w) to 0.02 ppt (w/w), 0.02 ppt (w/w) to 0.05 ppt (w/w), 0.05 ppt (w/w) to 0.1 ppt (w/w), 0.1 ppt (w/w) to 0.2 ppt (w/w), 0.2 ppt (w/w) to 0.5 ppt (w/w), 0.5 ppt (w/w) to 1 ppt (w/w), 1 ppt (w/w) to 2 ppt (w/w), 2 ppt (w/w) to 5 ppt (w/w), 5 ppt (w/w) to 10 ppt (w/w), 10 ppt (w/w) to 20 ppt (w/w), 20 ppt (w/w) to 50 ppt (w/w), 50 ppt (w/w) to 100 ppt (w/w), 100 ppt (w/w) to 200 ppt (w/w), 200 ppt (w/w) to 500 ppt (w/w), 500 ppt (w/w) to 1 ppb (w/w), 1 ppb (w/w) to 2 ppb (w/w), 2 ppb (w/w) to 5 ppb (w/w), 5 ppb (w/w) to 10 ppb (w/w), 10 ppb (w/w) to 20 ppb (w/w), 20 ppb (w/w) to 50 ppb (w/w), 50 ppb (w/w) to 100 ppb (w/w), 100 ppb (w/w) to 200 ppb (w/w), 200 ppb (w/w) to 500 ppb (w/w), 500 ppb (w/w) to 1 ppm (w/w), 1 ppm (w/w) to 2 ppm (w/w), 2 ppm (w/w) to 5 ppm (w/w), 5 ppm (w/w) to 10 ppm (w/w), 10 ppm (w/w) to 20 ppm (w/w), 20 ppm (w/w) to 50 ppm (w/w), or 50 ppm (w/w) to 100 ppm (w/w). The concentration of the active ingredient at the time of eating may specifically be, for example, 0.001 ppt (w/w) to 100 ppm (w/w), 0.01 ppt (w/w) to 100 ppm (w/w), 0.01 ppt (w/w) to 50 ppm (w/w), 0.01 ppt (w/w) to 20 ppm (w/w), 0.01 ppt (w/w) to 10 ppm (w/w), 0.1 ppt (w/w) to 100 ppm (w/w), 0.1 ppt (w/w) to 50 ppm (w/w), 0.1 ppt (w/w) to 20 ppm (w/w), 0.1 ppt (w/w) to 10 ppm (w/w), or 0.1 ppt (w/w) to 1 ppm (w/w).

The concentration of 2-thiophenethiol at the time of eating may be, for example, in the range of the concentration of the active ingredient at the time of eating exemplified above. The concentration of 2-thiophenethiol at the time of eating may also be, for example, 0.001 ppt (w/w) or higher, 0.002 ppt (w/w) or higher, 0.005 ppt (w/w) or higher, 0.01 ppt (w/w) or higher, 0.02 ppt (w/w) or higher, 0.05 ppt (w/w) or higher, 0.1 ppt (w/w) or higher, 0.2 ppt (w/w) or higher, 0.5 ppt (w/w) or higher, 1 ppt (w/w) or higher, 2 ppt (w/w) or higher, 5 ppt (w/w) or higher, 10 ppt (w/w) or higher, 20 ppt (w/w) or higher, 50 ppt (w/w) or higher, 100 ppt (w/w) or higher, 200 ppt (w/w) or higher, or 500 ppt (w/w) or higher, and may be 1 ppb (w/w) or lower, 500 ppt (w/w) or lower, 200 ppt (w/w) or lower, 100 ppt (w/w) or lower, 50 ppt (w/w) or lower, 20 ppt (w/w) or lower, 10 ppt (w/w) or lower, 5 ppt (w/w) or lower, 2 ppt (w/w) or lower, 1 ppt (w/w) or lower, 0.5 ppt (w/w) or lower, 0.2 ppt (w/w) or lower, 0.1 ppt (w/w) or lower, 0.05 ppt (w/w) or lower, 0.02 ppt (w/w) or lower, 0.01 ppt (w/w) or lower, 0.005 ppt (w/w) or lower, or 0.002 ppt (w/w) or lower, or may be in a range defined by any non-contradictory combination of these exemplary minimum and maximum concentrations. The concentration of 2-thiophenethiol at the time of eating may specifically be, for example, 0.001 ppt (w/w) to 1 ppb (w/w), 0.01 ppt (w/w) to 100 ppt (w/w), or 0.1 ppt (w/w) to 10 ppt (w/w).

The concentration of 2-phenylethanethiol, 2-phenyl-2-butenal, 2-thiophenemethanethiol, or S-(2-methyl-3-furyl) ethanethioate at the time of eating may be, for example, in the range of the concentration of the active ingredient at the time of eating exemplified above. The concentration of 2-phenylethanethiol, 2-phenyl-2-butenal, 2-thiophenemethanethiol, or S-(2-methyl-3-furyl) ethanethioate at the time of eating may also be, for example, 0.01 ppt (w/w) or higher, 0.02 ppt (w/w) or higher, 0.05 ppt (w/w) or higher, 0.1 ppt (w/w) or higher, 0.2 ppt (w/w) or higher, 0.5 ppt (w/w) or higher, 1 ppt (w/w) or higher, 2 ppt (w/w) or higher, 5 ppt (w/w) or higher, 10 ppt (w/w) or higher, 20 ppt (w/w) or higher, 50 ppt (w/w) or higher, 100 ppt (w/w) or higher, 200 ppt (w/w) or higher, 500 ppt (w/w) or higher, 1 ppb (w/w) or higher, 2 ppb (w/w) or higher, or 5 ppb (w/w) or higher, and may be 1 ppm (w/w) or lower, 500 ppb (w/w) or lower, 200 ppb (w/w) or lower, 100 ppb (w/w) or lower, 50 ppb (w/w) or lower, 20 ppb (w/w) or lower, 10 ppb (w/w) or lower, 5 ppb (w/w) or lower, 2 ppb (w/w) or lower, 1 ppb (w/w) or lower, 500 ppt (w/w) or lower, 200 ppt (w/w) or lower, 100 ppt (w/w) or lower, 50 ppt (w/w) or lower, 20 ppt (w/w) or lower, 10 ppt (w/w) or lower, 5 ppt (w/w) or lower, 2 ppt (w/w) or lower, 1 ppt (w/w) or lower, 0.5 ppt (w/w) or lower, 0.2 ppt (w/w) or lower, 0.1 ppt (w/w) or lower, 0.05 ppt (w/w) or lower, or 0.02 ppt (w/w) or lower, or may be in a range defined by any non-contradictory combination of these exemplary minimum and maximum concentrations. The concentration of 2-phenylethanethiol, 2-phenyl-2-butenal, 2-thiophenemethanethiol, or S-(2-methyl-3-furyl) ethanethioate at the time of eating may specifically be, for example, 0.01 ppt (w/w) to 1 ppm (w/w), 0.1 ppt (w/w) to 1 ppm (w/w), 1 ppt (w/w) to 1 ppm (w/w), 0.01 ppt (w/w) to 10 ppb (w/w), 0.1 ppt (w/w) to 1 ppb (w/w), or 1 ppt (w/w) to 100 ppt (w/w).

The concentration of diflurfuryl sulfide or 2-methylbenzenethiol at the time of eating may be, for example, in the range of the concentration of the active ingredient at the time of eating exemplified above. The concentration of diflurfuryl sulfide or 2-methylbenzenethiol at the time of eating may also be, for example, 0.1 ppt (w/w) or higher, 0.2 ppt (w/w) or higher, 0.5 ppt (w/w) or higher, 1 ppt (w/w) or higher, 2 ppt (w/w) or higher, 5 ppt (w/w) or higher 10 ppt (w/w) or higher, 20 ppt (w/w) or higher, 50 ppt (w/w) or higher, 100 ppt (w/w) or higher, 200 ppt (w/w) or higher, 500 ppt (w/w) or higher, 1 ppb (w/w) or higher, 2 ppb (w/w) or higher, 5 ppb (w/w) or higher, 10 ppb (w/w) or higher, 20 ppb (w/w) or higher, 50 ppb (w/w) or higher, 100 ppb (w/w) or higher, 200 ppb (w/w) or higher, or 500 ppb (w/w) or higher, and may be 100 ppb (w/w) or lower, 50 ppb (w/w) or lower, 20 ppb (w/w) or lower, 10 ppb (w/w) or lower, 5 ppb (w/w) or lower, 2 ppb (w/w) or lower, 1 ppb (w/w) or lower, 500 ppt (w/w) or lower, 200 ppt (w/w) or lower, 100 ppt (w/w) or lower, 50 ppt (w/w) or lower, 20 ppt (w/w) or lower, 10 ppt (w/w) or lower, 5 ppt (w/w) or lower, 2 ppt (w/w) or lower, 1 ppt (w/w) or lower, 0.5 ppt (w/w) or lower, or 0.2 ppt (w/w) or lower, or may be in a range defined by any non-contradictory combination of these exemplary minimum and maximum concentrations. The concentration of difurfuryl sulfide or 2-methylbenzenethiol at the time of eating may specifically be, for example, 0.1 ppt (w/w) to 100 ppb (w/w), 1 ppt (w/w) to 10 ppb (w/w), or 10 ppt (w/w) to 1 ppb (w/w).

The concentration of benzyl isothiocyanate, 4-methyl-1-phenyl-2-pentanol, or 2,4-heptadienal at the time of eating may be, for example, in the range of the concentration of the active ingredient exemplified above. The concentration of benzyl isothiocyanate, 4-methyl-1-phenyl-2-pentanol, or 2,4-heptadienal at the time of eating may also be, for example, 1 ppt (w/w) or higher, 2 ppt (w/w) or higher, 5 ppt (w/w) or higher, 10 ppt (w/w) or higher, 20 ppt (w/w) or higher, 50 ppt (w/w) or higher, 100 ppt (w/w) or higher, 200 ppt (w/w) or higher, 500 ppt (w/w) or higher, 1 ppb (w/w) or higher, 2 ppb (w/w) or higher, 5 ppb (w/w) or higher, 10 ppb (w/w) or higher, 20 ppb (w/w) or higher, 50 ppb (w/w) or higher, 100 ppb (w/w) or higher, 200 ppb (w/w) or higher, or 500 ppb (w/w) or higher, and may be 1 ppm (w/w) or lower, 500 ppb (w/w) or lower, 200 ppb (w/w) or lower, 100 ppb (w/w) or lower, 50 ppb (w/w) or lower, 20 ppb (w/w) or lower, 10 ppb (w/w) or lower, 5 ppb (w/w) or lower, 2 ppb (w/w) or lower, 1 ppb (w/w) or lower, 500 ppt (w/w) or lower, 200 ppt (w/w) or lower, 100 ppt (w/w) or lower, 50 ppt (w/w) or lower, 20 ppt (w/w) or lower, 10 ppt (w/w) or lower, 5 ppt (w/w) or lower, or 2 ppt (w/w) or lower, or may be in a range defined by any non-contradictory combination of these exemplary minimum and maximum concentrations. The concentration of benzyl isothiocyanate, 4-methyl-1-phenyl-2-pentanol, or 2,4-heptadienal at the time of eating may specifically be, for example, 1 ppt (w/w) to 1 ppm (w/w), 10 ppt (w/w) to 100 ppb (w/w), or 100 ppt (w/w) to 10 ppb (w/w).

The concentration of N-vanillylnonanamide at the time of eating may be, for example, in the range of the concentration of the active ingredient at the time of eating exemplified above. The concentration of N-vanillylnonanamide at the time of eating may also be, for example, 10 ppt (w/w) or higher, 20 ppt (w/w) or higher, 50 ppt (w/w) or higher, 100 ppt (w/w) or higher, 200 ppt (w/w) or higher, 500 ppt (w/w) or higher, 1 ppb (w/w) or higher, 2 ppb (w/w) or higher, 5 ppb (w/w) or higher, 10 ppb (w/w) or higher, 20 ppb (w/w) or higher, 50 ppb (w/w) or higher, 100 ppb (w/w) or higher, 200 ppb (w/w) or higher, 500 ppb (w/w) or higher, 1 ppm (w/w) or higher, 2 ppm (w/w) or higher, or 5 ppm (w/w) or higher, and 10 ppm (w/w) or lower, 5 ppm (w/w) or lower, 2 ppm (w/w) or lower, 1 ppm (w/w) or lower, 500 ppb (w/w) or lower, 200 ppb (w/w) or lower, 100 ppb (w/w) or lower, 50 ppb (w/w) or lower, 20 ppb (w/w) or lower, 10 ppb (w/w) or lower, 5 ppb (w/w) or lower, 2 ppb (w/w) or lower, 1 ppb (w/w) or lower, 500 ppt (w/w) or lower, 200 ppt (w/w) or lower, 100 ppt (w/w) or lower, 50 ppt (w/w) or lower, or 20 ppt (w/w) or lower, or may be in a range defined by any non-contradictory combination of these exemplary minimum and maximum concentrations. The concentration of N-vanillylnonanamide at the time of eating may specifically be, for example, 10 ppt (w/w) to 10 ppm (w/w), 100 ppt (w/w) to 1 ppm (w/w), or 1 ppb (w/w) to 100 ppb (w/w).

The concentration of nootkatone or benzyl isoeugenol at the time of eating may be, for example, in the range of the concentration of the active ingredient at the time of eating exemplified above. The concentration of nootkatone or benzyl isoeugenol at the time of eating may also be, for example, 100 ppt (w/w) or higher, 200 ppt (w/w) or higher, 500 ppt (w/w) or higher, 1 ppb (w/w) or higher, 2 ppb (w/w) or higher, 5 ppb (w/w) or higher, 10 ppb (w/w) or higher, 20 ppb (w/w) or higher, 50 ppb (w/w) or higher, 100 ppb (w/w) or higher, 200 ppb (w/w) or higher, 500 ppb (w/w) or higher, 1 ppm (w/w) or higher, 2 ppm (w/w) or higher, 5 ppm (w/w) or higher, 10 ppm (w/w) or higher, 20 ppm (w/w) or higher, or 50 ppm (w/w) or higher, and 100 ppm (w/w) or lower, 50 ppm (w/w) or lower, 20 ppm (w/w) or lower, 10 ppm (w/w) or lower, 5 ppm (w/w) or lower, 2 ppm (w/w) or lower, 1 ppm (w/w) or lower, 500 ppb (w/w) or lower, 200 ppb (w/w) or lower, 100 ppb (w/w) or lower, 50 ppb (w/w) or lower, 20 ppb (w/w) or lower, 10 ppb (w/w) or lower, 5 ppb (w/w) or lower, 2 ppb (w/w) or lower, 1 ppb (w/w) or lower, 500 ppt (w/w) or lower, or 200 ppt (w/w) or lower, or may be in a range defined by any non-contradictory combination of these exemplary minimum and maximum concentrations. The concentration of nootkatone or benzyl isoeugenol at the time of eating may specifically be, for example, 0.1 ppb (w/w) to 100 ppm (w/w), 1 ppb (w/w) to 100 ppm (w/w), 10 ppb (w/w) to 100 ppm (w/w), 1 ppb (w/w) to 10 ppm (w/w), or 10 ppb (w/w) to 1 ppm (w/w).

The concentration of the active ingredient at the time of eating may also be, for example, lower than the threshold concentration of the active ingredient. The concentration of the active ingredient at the time of eating may specifically be, for example, in the range of the concentration of the active ingredient at the time of eating exemplified above, and lower than the threshold concentration of the active ingredient. The term "threshold concentration of the active ingredient" means the maximum concentration of the active ingredient at which odor of the active ingredient itself is not felt when an aqueous solution containing the active ingredient alone is ingested. Examples of the threshold concentration of the active ingredient include the threshold concentrations described in the examples.

The description for the addition of the active ingredient can be applied to the addition of the composition of the present invention. For example, the composition of the present invention can be added so that the addition amount of the active ingredient exemplified above can be attained.

The ingredient other than the active ingredient may be added to a raw material of food so that, for example, the concentration of the ingredient other than the active ingredient at the time of eating should be in a desired range (e.g., the range shown below). When sclareol is used in combination with the active ingredient, the concentration of sclareol at the time of eating may be, for example, in the range of the concentration of the active ingredient at the time of eating exemplified above.

The food of the present invention may contain an ingredient that can generate 4-methylacetophenone (e.g., citral). That is, the food of the present invention may be produced to contain an ingredient that can generate 4-methylacetophenone. Such a food containing an ingredient that can generate 4-methylacetophenone can be produced by, for example, adding an ingredient that can generate 4-methylacetophenone. That is, the second embodiment of the method of the present invention may further comprise adding an ingredient that can generate 4-methylacetophenone to a raw material of food. Such a food containing an ingredient that can generate 4-methylacetophenone may be produced by, for example, adding an ingredient that can generate 4-methylacetophenone itself, or by adding a material containing an ingredient that can generate 4-methylacetophenone. As the ingredient that can generate 4-methylacetophenone, a commercial product may be used, or such an ingredient appropriately produced and obtained may be used. The method for producing the ingredient that can generate 4-methylacetophenone is not particularly limited. The ingredient that can generate 4-methylacetophenone can be produced by, for example, chemical synthesis, enzymatic reaction, fermentation, extraction, or a combination thereof. The addition of the ingredient that can generate 4-methylacetophenone can be carried out in the same manner as the addition of the active ingredient. The ingredient that can generate 4-methylacetophenone may be added to a raw material of food, for example, so that the content of the ingredient that can generate 4-methylacetophenone in the food of the present invention is in a desired range (e.g., the range of the content described below). The food containing the ingredient that can generate 4-methylacetophenone can also be produced by, for example, using a raw material of food containing the ingredient that can generate 4-methylacetophenone. That is, the raw material of food may contain the ingredient that can generate 4-methylacetophenone.

When the ingredient that can generate 4-methylacetophenone can form a salt, the ingredient that can generate 4-methylacetophenone may be used as a free form, a salt, or a combination thereof. That is, the term "ingredient that can generate 4-methylacetophenone" may mean an ingredient that can generate 4-methylacetophenone in the form of free form or a salt thereof, or a combination thereof, unless otherwise noted. The term "free form" means a form that does not form a salt. If the ingredient that can generate 4-methylacetophenone can form a hydrate, the ingredient that can generate 4-methylacetophenone may be used as anhydrate, hydrate, or a combination thereof. That is, the term "ingredient that can generate 4-methylacetophenone" (e.g., "the ingredient that can generate 4-methylacetophenone in the form of free form" or "salt of the ingredient that can generate 4-methylacetophenone") may encompass anhydrate and hydrate, unless otherwise noted. The ingredient that can generate 4-methylacetophenone may be in any form, such as in the form of ion, at the time of use.

The salt is not particularly limited as long as it can be orally ingested. For example, specific examples of salt for acidic group such as carboxyl group include ammonium salts, salts with alkali metals such as sodium and potassium, salts with alkaline earth metals such as calcium and magnesium, aluminum salts, zinc salts, salts with organic amines such as triethylamine, ethanolamine, morpholine, pyrrolidine, piperidine, piperazine, and dicyclohexylamine, and salts with basic amino acids such as arginine and lysine. Specific examples of salt for basic group such as amino group include salts with inorganic acids such as hydrochloric acid, sulfuric acid, phosphoric acid, nitric acid, and hydrobromic acid, salts with organic carboxylic acids such as acetic acid, citric acid, benzoic acid, maleic acid, fumaric acid, tartaric acid, succinic acid, tannic acid, butyric acid, hibenzic acid, pamoic acid, enanthic acid, decanoic acid, theoclic acid, salicylic acid, lactic acid, oxalic acid, mandelic acid, malic acid, methylmalonic acid, and adipic acid, and salts with organic sulfonic acids such as methanesulfonic acid, benzenesulfonic acid, and p-toluenesulfonic acid. As the salt, one type of salt may be used, or a combination of two or more types of salts may be used.

When a material containing the ingredient that can generate 4-methylacetophenone is used, the amount (e.g.,

content (concentration) or amount used) of the ingredient that can generate 4-methylacetophenone shall be calculated on the basis of the amount of the ingredient that can generate 4-methylacetophenone itself contained in the material. When the ingredient that can generate 4-methylacetophenone forms a salt or hydrate, the amount (e.g., content (concentration) or amount used) of the ingredient that can generate 4-methylacetophenone shall be calculated on the basis of the mass of equimolar amount of unhydrated free form corresponding to the salt or hydrate. The same shall apply to 4-methylacetophenone.

When the food of the present invention contains the ingredient that can generate 4-methylacetophenone (e.g., citral), the content of the ingredient that can generate 4-methylacetophenone in the food of the present invention is, as the concentration at the time of eating, for example, 100 ppb (w/w) or higher, 200 ppb (w/w) or higher, 500 ppb (w/w) or higher, 1 ppm (w/w) or higher, 2 ppm (w/w) or higher, 5 ppm (w/w) or higher, 10 ppm (w/w) or higher, 20 ppm (w/w) or higher, 50 ppm (w/w) or higher, 100 ppm (w/w) or higher, 200 ppm (w/w) or higher, or 500 ppm (w/w) or higher, and may be 1000 ppm (w/w) or lower, 500 ppm (w/w) or lower, 200 ppm (w/w) or lower, 100 ppm (w/w) or lower, 50 ppm (w/w) or lower, 20 ppm (w/w) or lower, 10 ppm (w/w) or lower, 5 ppm (w/w) or lower, 2 ppm (w/w) or lower, 1 ppm (w/w) or lower, 500 ppb (w/w) or lower, or 200 ppb (w/w) or lower, or may be in a range defined by any non-contradictory combination of these exemplary minimum and maximum concentrations. The content of the ingredient that can generate 4-methylacetophenone (e.g., citral) in the food of the present invention may specifically be, as the concentration at the time of eating, for example, 100 ppb (w/w) to 200 ppb (w/w), 200 ppb (w/w) to 500 ppb (w/w), 500 ppb (w/w) to 1 ppm (w/w), 1 ppm (w/w) to 2 ppm (w/w), 2 ppm (w/w) to 5 ppm (w/w), 5 ppm (w/w) to 10 ppm (w/w), 10 ppm (w/w) to 20 ppm (w/w), 20 ppm (w/w) to 50 ppm (w/w), 50 ppm (w/w) to 100 ppm (w/w), 100 ppm (w/w) to 200 ppm (w/w), 200 ppm (w/w) to 500 ppm (w/w), or 500 ppm (w/w) to 1000 ppm (w/w). The content of the ingredient that can generate 4-methylacetophenone (e.g., citral) in the food of the present invention may specifically be, as the concentration at the time of eating, for example, 100 ppb (w/w) to 1000 ppm (w/w). In addition, in one embodiment, the content of the ingredient that can generate 4-methylacetophenone in the food of the present invention exemplified above may be read as the total of the content of the ingredient that can generate 4-methylacetophenone and the content of 4-methylacetophenone in the food of the present invention.

The food of the present invention may contain 4-methylacetophenone. That is, the food of the present invention may be produced to contain 4-methylacetophenone. Such a food containing 4-methylacetophenone may be produced by, for example, adding 4-methylacetophenone. That is, the second embodiment of the method of the present invention may further comprise adding 4-methylacetophenone to a raw material of food. The food containing 4-methylacetophenone may be produced by, for example, adding 4-methylacetophenone itself, or adding a material containing 4-methylacetophenone, such as seasonings containing 4-methylacetophenone. As 4-methylacetophenone, a commercially available product may be used, or one appropriately produced and obtained may be used. The method for producing 4-methylacetophenone is not particularly limited. 4-Methylacetophenone may be produced by chemical synthesis, enzymatic reaction, fermentation, extraction, or a combination thereof. The addition of 4-methylacetophenone can be carried out in the same manner as the addition of the active ingredient. 4-Methylacetophenone can be added to a raw material of food so that, for example, the content of 4-methylacetophenone in the food of the present invention is within a desired range (e.g., the content range described below). The food containing 4-methylacetophenone can also be produced by, for example, using a raw material of food containing 4-methylacetophenone. That is, the raw material of food may contain 4-methylacetophenone. 4-Methylacetophenone may also be generated, for example, in the production process of the food of the present invention. 4-Methylacetophenone may be generated by, for example, heating an ingredient that can generate 4-methylacetophenone. Therefore, the second embodiment of the method of the present invention may comprise, for example, the step of heating a raw material of food or food containing an ingredient that can generate 4-methylacetophenone. The heating may be carried out before or after the addition of the active ingredient. 4-Methylacetophenone may also be generated, for example, after the production process of the food of the present invention is performed (e.g., during storage of the food of the present invention).

Conditions for the heating are not particularly limited as long as 4-methylacetophenone is generated. The heating temperature may be, for example, 50°C or higher, 60°C or higher, 70°C or higher, 80°C or higher, 90°C or higher, or 100°C or higher, and may be 200°C or lower, 150°C or lower, 120°C or lower, 100°C or lower, 90°C or lower, or 80°C or lower, or may be in a range defined by any non-contradictory combination of these exemplary minimum and maximum temperatures. The heating temperature may specifically be, for example, 50 to 200°C or 80 to 150°C. The heating time may be, for example, 1 minute or longer, 3 minutes or longer, 5 minutes or longer, 10 minutes or longer, 15 minutes or longer, 20 minutes or longer, 30 minutes or longer, or 60 minutes or longer, and 120 minutes or shorter, 90 minutes or shorter, 60 minutes or shorter, or 30 minutes or shorter, or may be in a range defined by any non-contradictory combination of these exemplary minimum and maximum durations. The heating time may specifically be, for example, 1 to 120 minutes, 10 to 90 minutes, or 30 to 60 minutes. Examples of the heating method include incubating, baking, steaming, boiling, and frying. The heating may be carried out, for example, in conjunction with carrying out the food production process, or separately from the food production process. Further, for example, a part of the heating may be carried out in conjunction with carrying out the food production process and the remainder may be carried out separately from the food production process. For example, if the production process of the food inherently comprises a heating step, the production process of the food may also serve as a part or all of the heating.

When the food of the present invention contains 4-methylacetophenone, the content of 4-methylacetophenone in the food of the present invention may be, for example, as the concentration at the time of eating, 1 ppb (w/w) or higher, 2 ppb (w/w) or higher, 5 ppb (w/w) or higher, 10 ppb (w/w) or higher, 20 ppb (w/w) or higher, 50 ppb (w/w) or higher, 100 ppb (w/w) or higher, 200 ppb (w/w) or higher, 500 ppb (w/w) or higher, 1 ppm (w/w) or higher, 2 ppm (w/w) or higher, 5 ppm (w/w) or higher, 10 ppm (w/w) or higher, 20 ppm (w/w) or higher, or 50 ppm (w/w) or higher, and may be 100 ppm (w/w) or lower, 50 ppm (w/w) or lower, 20 ppm (w/w) or lower, 10 ppm (w/w) or lower, 5 ppm (w/w) or lower, 2 ppm (w/w) or lower, 1 ppm (w/w) or lower, 500 ppb (w/w) or lower, 200 ppb (w/w) or lower, 100 ppb (w/w) or lower, 50 ppb (w/w) or lower, 20 ppb (w/w) or lower, 10 ppb (w/w) or lower, 5 ppb (w/w) or lower, or 2 ppb (w/w) or lower, or may be in a range defined by any non-contradictory combination of these exemplary minimum and maximum contents. The content of 4-methylacetophenone in the food of the present invention may specifically be, as the concentration at the time of eating, for example, 1 ppb (w/w) to 2 ppb (w/w), 2 ppb (w/w) to 5 ppb (w/w), 5 ppb (w/w) to 10 ppb (w/w), 10 ppb (w/w) to 20 ppb (w/w), 20 ppb (w/w) to 50 ppb (w/w), 50 ppb (w/w) to 100 ppb (w/w), 100 ppb (w/w) to 200 ppb (w/w), 200 ppb (w/w) to 500 ppb (w/w), 500 ppb (w/w) to 1 ppm (w/w), 1 ppm (w/w) to 2 ppm (w/w), 2 ppm (w/w) to 5 ppm (w/w), 5 ppm (w/w) to 10 ppm (w/w), 10 ppm (w/w) to 20 ppm (w/w), 20 ppm (w/w) to 50 ppm (w/w), or 50 ppm (w/w) to 100 ppm (w/w). The content of 4-methylacetophenone in the food of the present invention may specifically be, as the concentration at the time of eating, for example, 1 ppb (w/w) to 100 ppm (w/w), 10 ppb (w/w) to 10 ppm (w/w), or 50 ppb (w/w) to 2 ppm (w/w).

<2-4> Use of active ingredient

The present invention also discloses use of the active ingredient for the purposes exemplified above. That is, the present invention discloses, for example, use of the active ingredient for suppressing 4-methylacetophenone odor of a food or for producing a food, or use of the active ingredient for producing a composition for suppressing 4-methylacetophenone odor of a food or production of a food.

The present invention also discloses the active ingredient for use for the purposes exemplified above. That is, the present invention discloses, for example, the active ingredient for use in suppression of 4-methylacetophenone odor of a food or for producing a food, and the active ingredient for use in production of a composition for suppressing 4-methylacetophenone odor of a food or for producing a food.

EXAMPLES

Hereafter, the present invention will be explained in more detail with reference to non-limiting examples.

Unless otherwise noted, the raw materials, reagents, compounds and so forth used in the examples are readily obtained or prepared according to methods normally practiced in the art, or commercially available.

Example 1: Screening for substance that suppresses 4-methylacetophenone odor using olfactory receptors

<1> Preparation of human olfactory receptor-expressing cells

<1-1> Preparation of expression vectors for human olfactory receptors

As olfactory receptors, 352 types of human olfactory receptors (OR1A1, OR1A2, OR1B1, OR1C1, OR1D2, OR1D5, OR1E1, OR1F1, OR1F12, OR1G1, OR1I1, OR1J1, OR1J2, OR1J4, OR1K1, OR1L1, OR1L3, OR1L4, OR1L8, OR1M1, OR1N1, OR1N2, OR1Q1, OR1R1P, OR1S1, OR2A1, OR2A2, OR2A4, OR2A5, OR2A12, OR2A14, OR2A25, OR2AE1, OR2AG1, OR2AG2, OR2AJ1P, OR2AK2, OR2AP1, OR2AT4, OR2B2, OR2B3, OR2B6, OR2B11, OR2C1, OR2C3, OR2D2, OR2D3, OR2F1, OR2G2, OR2G3, OR2G6, OR2H1, OR2H2, OR2J2, OR2J3, OR2K2, OR2L2, OR2L8, OR2L13, OR2M2, OR2M4, OR2M7, OR2S2, OR2T1, OR2T2, OR2T5, OR2T6, OR2T8, OR2T10, OR2T11, OR2T27, OR2T34, OR2V2, OR2W1, OR2W3, OR2Y1, OR2Z1, OR3A1, OR3A2, OR3A3, OR3A4, OR4A5, OR4A15, OR4A16, OR4A47, OR4B1, OR4C3, OR4C5, OR4C6, OR4C11, OR4C12, OR4C13, OR4C15, OR4C16, OR4C46, OR4D1, OR4D2, OR4D5, OR4D6, OR4D9, OR4D10, OR4D11, OR4E2, OR4F3, OR4F5, OR4F6, OR4F14P, OR4F15, OR4G11P, OR4H12P, OR4K1, OR4K2, OR4K5, OR4K13, OR4K14, OR4K15, OR4K17, OR4L1, OR4M1, OR4N2, OR4N4, OR4N5, OR4P4, OR4Q3, OR4S1, OR4S2, OR4X1, OR4X2, OR5A1, OR5A2, OR5AC2, OR5AK2, OR5AK3P, OR5AN1, OR5AP2, OR5AR1, OR5AS1, OR5B2, OR5B3, OR5B12, OR5B17, OR5B21, OR5C1, OR5D13, OR5D14, OR5D16, OR5D18, OR5F1, OR5H1, OR5H2, OR5H6, OR5H14, OR5I1, OR5J2, OR5K1, OR5K3, OR5K4, OR5L2, OR5M3, OR5M8, OR5M9, OR5M10, OR5M11, OR5P3, OR5R1, OR5T1, OR5T2, OR5T3, OR5V1, OR5W2, OR6A2, OR6B1, OR6B2, OR6C1, OR6C2, OR6C3, OR6C4, OR6C6, OR6C65, OR6C66P, OR6C68, OR6C70, OR6C74, OR6C75, OR6C76, OR6F1, OR6J1, OR6K2, OR6K3, OR6K6, OR6M1, OR6N1, OR6N2, OR6P1, OR6Q1, OR6S1, OR6T1, OR6V1, OR6X1, OR6Y1, OR7A3P, OR7A5, OR7A10, OR7A17, OR7C1, OR7C2, OR7D2, OR7D4, OR7E24, OR7G1, OR7G2, OR7G3, OR8A1, OR8B3, OR8B4, OR8B8, OR8B12, OR8D1, OR8D2, OR8D4, OR8G2, OR8G5, OR8H3, OR8I2, OR8J1, OR8J3, OR8K1, OR8K3, OR8K5, OR8S1, OR8U1, OR9A4, OR9G1, OR9G4, OR9I1, OR9K2, OR9Q1, OR9Q2, OR10A3, OR10A4, OR10A5, OR10A6, OR10A7, OR10AD1, OR10AG1, OR10C1, OR10D3, OR10D4P, OR10G2, OR10G3, OR10G4, OR10G6, OR10G7, OR10G9, OR10H2, OR10H4, OR10J1, OR10J3, OR10J5, OR10K1, OR10K2, OR10P1, OR10Q1, OR10R2, OR10S1, OR10T2, OR10V1, OR10W1, OR10X1, OR10Z1, OR11A1, OR11G2, OR11H4, OR11H6, OR11H12, OR11L1, OR12D2, OR12D3, OR13A1, OR13C2, OR13C3, OR13C4, OR13C8, OR13D1, OR13F1, OR13G1, OR13H1, OR13J1, OR14A2, OR14A16, OR14C36, OR14I1, OR14J1, OR14K1, OR14L1P, OR51A1P, OR51A4, OR51A7, OR51B2, OR51B4, OR51B5, OR51B6, OR51D1, OR51E1, OR51E2, OR51F1, OR51F2, OR51F5P, OR51G1, OR51G2, OR51H1, OR51I1, OR51I2, OR51L1, OR51M1, OR51Q1, OR51S1, OR51T1, OR51V1, OR52A1, OR52A4, OR52A5, OR52B2, OR52B4, OR52B6, OR52D1, OR52E2, OR52E4, OR52E5, OR52E8, OR52H1, OR52I2, OR52J3, OR52K2, OR52L2P, OR52M1, OR52N1, OR52N2, OR52N4, OR52N5, OR52P2P, OR52R1, OR52W1, OR52Z1P, OR56A1, OR56A3, OR56A4, OR56A5, OR56B1, OR56B2P, and OR56B4) were employed.

The genes for the 352 types of human olfactory receptors were purchased from TrueClone cDNA Clone Collection (OriGene). Fragments for subcloning of the 352 types of human olfactory receptor genes were amplified by PCR using primers designed on the basis of sequence information available in GenBank and the purchased human olfactory receptor genes as the templates. Each of the amplified fragments for subcloning of the genes was subcloned into the Rho-pME18S vector (K. Kajiya et al., Journal of Neuroscience. 15 August 2001, 21 (16) 6018-6025) downstream of the Rho tag sequence using the EcoRI and XhoI sites to obtain 352 types of expression vectors for human olfactory receptors.

<1-2> Preparation of olfactory receptor-expressing cells

HEK293T cells expressing each of the 352 types of olfactory receptors were prepared by the following procedure. An expression vector mixture having the composition shown in Table 1 was prepared. pcDNA3.1-microbat RTP1s is an expression vector for bat RTP 1s, pcDNA3.1 -Golf is an expression vector for human Golf, and pcDNA3.1-Ric8B is an expression vector for rat Ric8B (Japanese Patent Publication No. 2019-037197). The expression vector mixture was allowed to stand for 20 minutes in a laminar flow cabinet and then added to HEK293T cells inoculated in 10-cm petri dishes the day before (2.5 to $3.5 \times 10^6$ cells/10-cm petri dish). After incubation for 5 hours in an incubator maintained at 37°C and 5% $CO_2$, the HEK293T cells were inoculated into each well of a 96-well plate (BD) in a volume of 100 $\mu$l ($2.5 \times 10^6$ cells/ml), and cultured overnight in an incubator maintained at 37°C and 5% $CO_2$. As described above, cultures of the HEK293T cells expressing each of the 352 olfactory receptors were obtained. As a control, the same procedure was performed by using the empty vector Rho-pME18S instead of the expression vector for human olfactory receptor in the expression vector mixture to obtain culture of the HEK293T cells transfected with the empty vector Rho-pME18S (hereinafter also referred to as the "control cells").

[Table 1]

| Table 1: Composition of expression vector mixture | |
| --- | --- |
| OPTI-MEM (GIBCO) | 2.4 ml |
| Expression vector for human olfactory receptor | 0.8 $\mu$g |
| pGL4.29[luc2P/CRE/Hygro] Vector, Promega | 0.16 $\mu$g |
| pGL4.74[hRluc/TK] Vector, Promega | 0.08$\mu$g |
| pcDNA3.1-microbat RTP1s | 0.16$\mu$g |
| pcDNA3.1-Golf | 0.08$\mu$g |
| pcDNA3.1-Ric8B | 0.08$\mu$g |
| Lipofectamine 2000 (Invitrogen) | 27.2 $\mu$l |

<2> Luciferase assay

The olfactory receptors expressed by HEK293T cells conjugate with Golf to activate adenylate cyclase, and thereby increase the amount of intracellular cAMP. In this example, the olfactory receptor response was measured by using a luciferase reporter gene assay, in which the increase in intracellular cAMP amount is monitored as an increase in firefly luciferase-derived luminescence. The "luciferase reporter gene assay" is also referred to as the "luciferase assay". The firefly luciferase is expressed from the firefly luciferase gene carried by the pGL4.29[luc2P/CRE/Hygro] vector in an intracellular cAMP level-dependent manner. In addition, value of the luminescence derived from Renilla luciferase was used as an internal standard to correct errors caused by differences in gene transfer efficiency and cell number in each well. The Renilla luciferase is constitutively expressed from the Renilla luciferase gene carried by the pGL4.74[hRluc/TK] vector under the control of the CMV promoter.

The medium was removed from the cultures obtained in the section <1-2> mentioned above, and 60 μL of a 4-methylacetophenone solution (containing 300 μM or 30 μM of 4-methylacetophenone) was added to each to obtain reaction solutions. The 4-methylacetophenone solution was prepared by dissolving 4-methylacetophenone in CD293 (Life Technologies, Inc.). The reaction solution was placed in an incubator maintained at 37°C and 5% $CO_2$, and the cells were incubated for 3 hours, so that the firefly luciferase gene was fully expressed in the cells. The value of luminescence derived from the firefly luciferase in the cells was measured and designated as "Luc value". The value of luminescence derived from Renilla luciferase in the cells was also measured and designated as "hRLuc value". The value of luminescence derived from each luciferase was measured by using Dual-Glo™ Luciferase Assay System (Promega) according to the operation manual of the product.

The value of luminescence derived from firefly luciferase induced by stimulation with 4-methylacetophenone (Luc value) was divided by the value of luminescence derived from Renilla luciferase (hRluc value) obtained for the same well to obtain "Luc/hRluc value". The Luc/hRluc value of the cells stimulated with 4-methylacetophenone was divided by the Luc/hRluc value of the cells not stimulated with 4-methylacetophenone stimulation to obtain "fold increase". Further, the fold increase of the cells transfected with the olfactory receptor expression vector was divided by the fold increase of the control cells (cells transfected with the empty vector Rho-pME18S) to obtain "normalized response". The common logarithm of the normalized response was used as the "olfactory receptor activity", a quantitative index of the intensity of the response of the olfactory receptor to 4-methylacetophenone.

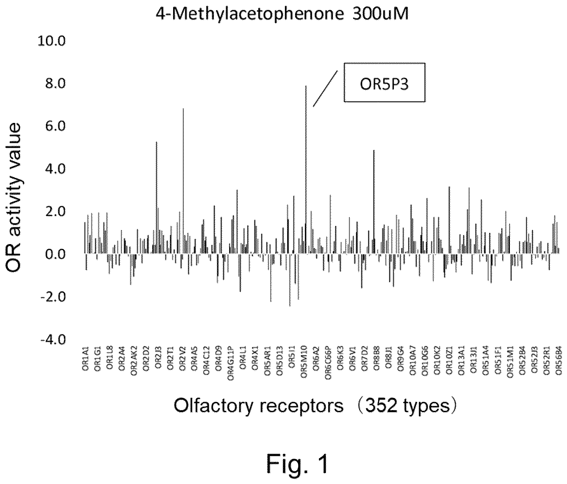

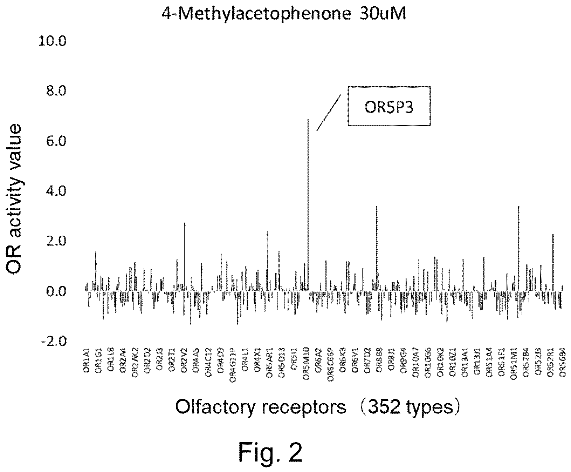

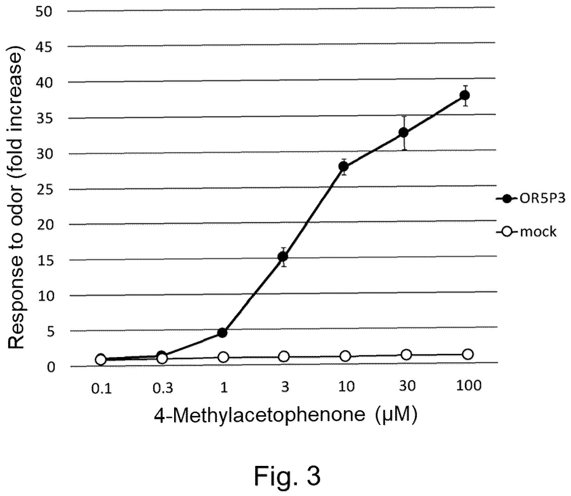

The results are shown in Figs. 1 to 3. The response to 300 μM and 30 μM 4-methylacetophenone was measured for the 352 types of olfactory receptors, and as a result, OR5P3 showed a response (Figs. 1 and 2). OR5P3 responded to 4-methylacetophenone in a concentration-dependent manner (Fig. 3). OR5P3 is a novel 4-methylacetophenone receptor that has not been found to respond to 4-methylacetophenone.

<3> Screening for OR5P3 antagonist

OR5P3 is known as a 2-heptanone receptor (Japanese Patent Publication No. 2019-129772). Therefore, screening for OR5P3 antagonist was performed by using 2-heptanone as a receptor-activating substance.

The medium was removed from the culture of HEK293T cells expressing OR5P3 obtained in the section <1-2> mentioned above, and 60 μl of a mixed solution of a test substance and 2-heptanone (containing 100 μM test substance and 1000 μM 2-heptanone) or 60 μl of a 2-heptanone solution (containing 1000 μM 2-heptanone) was added to obtain a reaction solution. The solutions were prepared by dissolving the substances in CD293 (Life Technologies, Inc.). The cells were incubated in a $CO_2$ incubator at 37°C for 3 hours so that the firefly luciferase gene was sufficiently expressed in the cells. The value of luminescence derived from firefly luciferase in the cells was measured and designated as "Luc value". The value of luminescence derived from firefly luciferase was measured by using Dual-Glo™ Luciferase Assay System (Promega) according to the operation manual of the product.

Receptor response inhibition ratio of the test substance was calculated as follows. The Luc value (Y) in the cells not stimulated with 2-heptanone was subtracted from the Luc value (X) in the cells stimulated with 2-heptanone alone. Similarly, the Luc value (Y) in the cells not stimulated with 2-heptanone was subtracted from the Luc value (Z) in the cells stimulated with a mixture of 2-heptanone and the test substance. The receptor response inhibition ratio of the test substance was calculated in accordance with the following equation using the increase in value of luminescence caused by stimulation with 2-heptanone alone (X - Y) as the standard. The experiment was performed in duplicate, and an average value was obtained.

$$\text{Inhibition ratio } (\%) = \{1 - (Z - Y)/(X - Y)\} \times 100$$

The test substances that showed receptor response inhibition activity were examined for concentration dependency of the inhibition activity. The concentration of the test substance was 0.3, 1, 3, 10, 30, or 100 μM. The response of the receptor to 2-heptanone (1000 μM) in the presence of each concentration of the test substance was measured and represented as the relative response intensity based on the response intensity of the receptor to 2-heptanone (1000 μM) observed in the absence of the test substance, which was taken as 100%. From the results, 50% inhibition concentration of each test substance ($IC_{50}$ value, μM) was calculated. The results are shown in Table 2.

[Table 2]

Table 2

| Compound | IC$_{50}$ ($\mu$M) |
|---|---|
| N-Vanillylnonanamide | 2 |
| Nootkatone | 8.1 |
| 2-Phenylethanethiol | 17 |
| Benzyl isothiocyanate | 19 |
| 2-Thiophenemethanethiol | 39 |
| Difurfuryl sulfide | 39 |
| 2-Methylbenzenethiol | 46 |
| Benzyl isoeugenol | 49 |
| 2-Phenyl-2-butenal | 65 |
| 4-Methyl-1 -phenyl-2-pentanol | 67 |
| 2-Thiophenethiol | 76 |
| 2,4-Heptadienal | 78 |
| S-(2-Methyl-3-furyl) ethanethioate | 45 |

Example 2: Evaluation of 4-methylacetophenone odor-masking effect

In this example, the 13 types of compounds that showed olfactory receptor response inhibition activity in Example 1 were evaluated for 4-methylacetophenone odor-masking effect.

[Test Example 1]

(Preparation of evaluation samples)

Each of the compounds shown in Table 3 was added to a 0.5 ppm 4-methylacetophenone aqueous solution to prepare each evaluation sample. The concentration of each compound added was the concentration at which odor of the compound itself is not felt when the aqueous solution containing the compound alone is put into the mouth and swallowed (threshold concentration).

(Evaluation of 4-methylacetophenone odor-masking effect)

The 4-methylacetophenone odor intensity of each evaluation sample was evaluated by putting 5 ml of the sample into the mouth and naturally swallowing it. The 4-methylacetophenone odor intensity was scored as 0 to 5 on the basis of the following evaluation criteria using 4-methylacetophenone aqueous solutions prepared at 0.5 ppm, 0.25 ppm, or 0 ppm as the standards. The evaluation was performed by a panel of 3 experts, and an average value of the results was calculated for each evaluation sample. On the basis of the 4-methylacetophenone odor intensity, the 4-methylacetophenone odor-masking effect was evaluated.

[Evaluation criteria for 4-methylacetophenone odor intensity]

0: 4-Methylacetophenone odor intensity of 0 ppm 4-methylacetophenone aqueous solution (water not containing 4-methylacetophenone)
3: 4-Methylacetophenone odor intensity of 0.25 ppm 4-methylacetophenone aqueous solution
5: 4-Methylacetophenone odor intensity of 0.5 ppm 4-methylacetophenone aqueous solution

[Evaluation criteria for 4-methylacetophenone odor-masking effect]

-: 4-Methylacetophenone odor intensity not lower than 5.2

±: 4-Methylacetophenone odor intensity not lower than 4.8 and lower than 5.2

+: 4-Methylacetophenone odor intensity not lower than 4.0 and lower than 4.8

++: 4-Methylacetophenone odor intensity not lower than 3 and lower than 4

+++: 4-Methylacetophenone odor intensity lower than 3 (intensity equivalent to that of 0.25 ppm 4-methylacetophenone aqueous solution)

The results are shown in Table 3. All of the evaluated compounds exhibited 4-methylacetophenone odor-masking effect.

Separately, 4-methylacetophenone odor-masking effect was also confirmed for each of the compounds shown in Table 3 by using a commercial lemon juice-containing beverage that was degraded to present 4-methylacetophenone odor (data not shown).

[Table 3]

Table 3

| Compound | Addition amount | Relative concentration based on threshold concentration | Masking effect |
|---|---|---|---|
| N-Vanillylnonanamide | 10ppb | Threshold | ++ |
| Nootkatone | 100ppb | Threshold | ++ |
| 2-Phenylethanethiol | 10ppt | Threshold | + |
| Benzyl isothiocyanate | 1ppb | Threshold | ++ |
| 2-Thiophenemethanethiol | 10ppt | Threshold | ++ |
| Difurfuryl sulfide | 100ppt | Threshold | ++ |
| 2-Methylbenzenethiol | 100ppt | Threshold | + |
| Benzyl isoeugenol | 100ppb | Threshold | ++ |
| 2-Phenyl-2-butenal | 10ppt | Threshold | ++ |
| 4-Methyl-1 -phenyl-2-pentanol | 1ppb | Threshold | + |
| 2-Thiophenethiol | 1ppt | Threshold | + |
| 2,4-Heptadienal | 1ppb | Threshold | ++ |
| S-(2-Methyl-3-furyl) ethanethioate | 10ppt | Threshold | ++ |

[Test Example 2]

(Preparation of evaluation samples)

Evaluation samples were prepared by adding each of the compounds shown in Table 4 to a 0.5 ppm 4-methylacetophenone aqueous solution. The concentration of each compound added was set at 1/10 of the threshold concentration, threshold concentration, or 10 times of the threshold concentration. The threshold concentration is the concentration at which odor of the compound itself is not felt when an aqueous solution containing each compound alone is put into the mouth and swallowed.

(Evaluation of 4-methylacetophenone odor-masking effect)

The 4-methylacetophenone odor intensity of each evaluation sample was evaluated by putting 5 ml of the sample into the mouth and naturally swallowing it. The 4-methylacetophenone odor intensity was scored as 0 to 10 on the basis of the following evaluation criteria using 4-methylacetophenone aqueous solutions prepared at 0.5 ppm, 0.25 ppm, and 0 ppm as the standards. The evaluation was performed by a panel of 6 experts, and an average value of the results was calculated for each evaluation sample. On the basis of the 4-methylacetophenone odor intensity, the 4-methylacetophenone odor-masking effect was evaluated.

[Evaluation criteria for 4-methylacetophenone odor intensity]

0: 4-Methylacetophenone odor intensity of 0 ppm 4-methylacetophenone aqueous solution (water not containing 4-methylacetophenone)
3: 4-Methylacetophenone odor intensity of 0.25 ppm 4-methylacetophenone aqueous solution
5: 4-Methylacetophenone odor intensity of 0.5 ppm 4-methylacetophenone aqueous solution

[Evaluation criteria for 4-methylacetophenone odor-masking effect]

-: 4-Methylacetophenone odor intensity not lower than 5.2
±: 4-Methylacetophenone odor intensity not lower than 4.8 and lower than 5.2
+: 4-Methylacetophenone odor intensity not lower than 4.0 and lower than 4.8
++: 4-Methylacetophenone odor intensity not lower than 3 and lower than 4
+++: 4-Methylacetophenone odor intensity lower than 3 (intensity equivalent to that of 0.25 ppm 4-methylacetophenone aqueous solution)

The results are shown in Table 4. The evaluated compounds showed 4-methylacetophenone odor-masking effect at all the addition concentrations.

[Table 4]

Table 4

| Compound | Addition amount | Relative concentration based on threshold concentration | Masking effect |
|---|---|---|---|
| Nootkatone | 10ppb | 1/10 Of threshold | + |
| | 100ppb | Threshold | + |
| | 1 ppm | 10 Times of threshold | + |
| S-(2-Methyl-3-furyl) ethanethioate | 1ppt | 1/10 Of threshold | ++ |
| | 10ppt | Threshold | ++ |
| | 100ppt | 10 Times of threshold | ++ |
| 2-Thiophenethiol | 0.1 ppt | 1/10 Of threshold | ++ |
| | 1ppt | Threshold | + |
| | 10ppt | 10 Times of threshold | + |
| 4-Methyl-1-phenyl-2-pentanol | 100ppt | 1/10 Of threshold | + |
| | 1ppb | Threshold | + |
| | 10ppb | 10 Times of threshold | ++ |

[Test Example 3]

(Preparation of evaluation samples)

A 100 ppm citral aqueous solution (stored under refrigeration after preparation) was used as a normal sample. This solution was stored at 44°C for two weeks to generate a degraded odor derived from citral, and was used as a degraded sample. Evaluation samples were prepared by adding each of the compounds shown in Table 5 to the degraded sample at the concentrations shown in the same.

(Evaluation of citral-derived degradation odor-masking effect)

The citral-derived degradation odor intensity of each evaluation sample was evaluated by putting 5 ml of the sample into the mouth and naturally swallowing it. The citral-derived degradation odor intensity was scored as 0 to 10 on the basis of the following evaluation criteria. The evaluation was performed by a panel of two experts, and an average value of the results was calculated for each evaluation sample. On the basis of the citral-derived degradation odor intensity, the citral-derived degradation odor-masking effect was evaluated.

[Evaluation criteria for citral-derived degradation odor intensity]

0: Citral-derived degradation odor intensity of the normal sample
5: Citral-derived degradation odor intensity of a mixture of the normal sample and degraded sample (normal sample degraded sample = 1: 1)
10: Citral-derived degradation odor intensity of the degraded sample

The results are shown in Table 5. All of the evaluated compounds showed citral-derived degradation odor-masking effect.

[Table 5]

Table 5

| Compound | Concentration of addition (ppm) | Evaluation score | Comment |
|---|---|---|---|
| trans, trans-2,4-Decadienal | 0.002 | 5 | Initial and middle degradation odors were suppressed. |
| trans, trans-2,4-Nonadienal | 0.002 | 7 | Initial and middle degradation odors were suppressed. |
| 2-Methylbenzenethiol | 0.165 | 8 | Initial, middle and after degradation odors were suppressed. |
| S-(2-Methyl-3-furyl) ethanethioate | 0.120 | 6 | Initial and middle degradation odors and middle and after browning odors were suppressed. |
| Piperine | 6.667 | 6 | Initial and middle degradation odors were suppressed. |
| Nootkatone | 10.000 | 5.5 | Initial, middle and after degradation odors were suppressed. |
| Sclareol | 16.667 | 5.5 | Initial, middle and after degradation odors were suppressed. |

[Test Example 4]

(Preparation of evaluation samples)

A commercially available lemon beverage (Chelate Lemon W Lemon (Pokka Sapporo), stored under refrigeration after purchased) was used as a normal sample. This lemon beverage was stored at 44°C for 2 weeks to generate a lemon-derived degradation odor and was used as a degraded sample. Evaluation samples were prepared by adding each of the compounds shown in Table 6 to the degraded sample at the concentrations shown in the same.

(Evaluation of lemon-derived degradation odor-masking effect)

The lemon-derived degradation odor intensity of each evaluation sample was evaluated by putting 5 ml of the sample into the mouth and naturally swallowing it. The lemon-derived degradation odor intensity was scored as 0 to 10 on the basis of the following evaluation criteria. The evaluation was performed by a panel of 2 experts, and an average value of the results was calculated for each evaluation sample. On the basis of the lemon-derived degradation odor intensity, the lemon-derived degradation odor-masking effect was evaluated.

[Evaluation criteria for lemon-derived degradation odor intensity]

0: Lemon-derived degradation odor intensity of the normal sample
5: Lemon-derived degradation odor intensity of a mixture of the normal sample and degraded sample (normal sample:degraded sample = 1: 1)
10: Lemon-derived degradation odor intensity of the degraded sample

The results are shown in Table 6. All of the evaluated compounds showed lemon-derived degradation odor-masking effect.

[Table 6]

Table 6

| Compound | Concentration of addition (ppm) | Evaluation score | Comment |
|---|---|---|---|
| trans, trans-2,4-Decadienal | 0.002 | 6 | Initial and middle degradation odors were suppressed. |
| trans, trans-2,4-Nonadienal | 0.002 | 7.5 | Initial and middle degradation odors were suppressed. |
| 2-Methylbenzenethiol | 0.165 | 8 | Initial, middle and after degradation odors were suppressed. |
| S-(2-Methyl-3-furyl) ethanethioate | 0.120 | 6 | Initial and middle degradation odors and middle and after browning odors were suppressed. |
| Piperine | 6.667 | 8 | Initial and middle degradation odors were suppressed. |
| Nootkatone | 10.000 | 6 | Initial, middle and after degradation odors were suppressed. |
| Sclareol | 16.667 | 6 | Initial, middle and after degradation odors were suppressed. |

[Test Example 5]

(Preparation of evaluation samples)

A commercially available lemon beverage (Chelate Lemon W Lemon (Pokka Sapporo), stored under refrigeration after purchased) was used as a normal sample. This lemon beverage was stored at 44°C for 2 weeks to generate a lemon-derived degradation odor and used as a degraded sample. Evaluation samples were prepared by adding the combination of each of the compounds shown in Table 7 to the degraded sample at the concentrations shown in the same.

(Evaluation of lemon-derived degradation odor-masking effect)

The lemon-derived degradation odor intensity of each evaluation sample was evaluated by putting 5 ml of the sample into the mouth and naturally swallowing it. The lemon-derived degradation odor intensity was scored as 0 to 10 on the basis of the following evaluation criteria. The evaluation was performed by a panel of 2 experts, and an average value of the results was calculated for each evaluation sample. On the basis of the lemon-derived degradation odor intensity, the lemon-derived degradation odor-masking effect was evaluated.

[Evaluation criteria for lemon-derived degradation odor intensity]

0: Lemon-derived degradation odor intensity of the normal sample
5: Lemon-derived degradation odor intensity of a mixture of the normal sample and degraded sample (normal sample:degraded sample = 1: 1)
10: Lemon-derived degradation odor intensity of the degraded sample

The results are shown in Table 7. All of the evaluated combinations of compounds showed lemon-derived degradation odor-masking effect.

[Table 7]

Table 7

| Compound | Concentration of addition (ppm) | |
|---|---|---|
| | Sample 1 | Sample 2 |
| S-(2-Methyl-3-furyl) ethanethioate | 0.12 | - |
| Nootkatone | - | 10.00 |
| Sclareol | 16.67 | 16.67 |
| Evaluation score | 5.5 | 5.5 |
| Comment | Initial and middle degradation odors were suppressed. | Initial and middle degradation odors were suppressed. |

INDUSTRIAL APPLICABILITY

According to the present invention, in one embodiment, substances that suppress 4-methylacetophenone odor can be efficiently screened for. In addition, according to the present invention, in one embodiment, 4-methylacetophenone odor can be suppressed.

<Explanation of Sequence Listing>

SEQ ID NO: 1, nucleotide sequence of human OR5P3 gene
SEQ ID NO: 2, amino acid sequence of human OR5P3 protein

**Claims**

1. A method for screening for a substance that suppresses 4-methylacetophenone odor, comprising the following steps (A) to (C):

(A) the step of contacting an olfactory receptor with an olfactory receptor-activating substance in the presence of a test substance;
(B) the step of measuring a response of the olfactory receptor to the olfactory receptor-activating substance; and
(C) the step of identifying the test substance as a substance that suppresses 4-methylacetophenone odor on the basis of the response; and wherein

when the response is inhibited by the test substance, the test substance is identified as a substance that suppresses 4-methylacetophenone odor, and

the olfactory receptor is OR5P3.

2. The method according to claim 1, wherein the response is activation of the olfactory receptor.

3. The method according to claim 1 or 2, wherein the olfactory receptor is used in the form of being carried by a cell, cell membrane, artificial lipid bilayer vesicle membrane, or artificial lipid bilayer membrane.

4. The method according to any one of claims 1 to 3, wherein the olfactory receptor is used in the form of being carried by a cell.

5. The method according to claim 3 or 4, wherein the cell is an animal cell.

6. The method according to any one of claims 1 to 5, wherein the steps (B) and (C) are carried out by the following steps (B1) and (C1), respectively:

(B1) the step of measuring degree of activation D1 of the olfactory receptor observed when the step (A) is performed; and

(C1) the step of identifying the test substance as a substance that suppresses 4-methylacetophenone odor on the basis of the degree of activation D1.

7. The method according to claim 6, wherein the step (C1) is carried out by the following step (C2):

(C2) the step of identifying the test substance as a substance that suppresses 4-methylacetophenone odor on the basis of difference between the degree of activation D1 and degree of activation D2 of the olfactory receptor observed under a control condition.

8. The method according to claim 7, wherein the control condition is the following condition (C2-1) or (C2-2):

(C2-1) a condition under which the olfactory receptor is contacted with the olfactory receptor-activating substance in the absence of the test substance; or

(C2-2) a condition under which the olfactory receptor is contacted with the olfactory receptor-activating substance in the presence of the test substance, and concentration of the test substance is lower than concentration of the test substance used in the step (A).

9. The method according to claim 7 or 8, wherein the method further comprises the step of measuring the degree of activation D2.

10. The method according to any one of claims 7 to 9, wherein the test substance is identified as a substance that suppresses 4-methylacetophenone odor, if the degree of activation D1 is lower than the degree of activation D2.

11. The method according to any one of claims 7 to 10, wherein the test substance is identified as a substance that suppresses 4-methylacetophenone odor, if the ratio of the degree of activation D1 to the degree of activation D2 is lower than 60%.

12. The method according to any one of claims 3 to 11, wherein the response is measured by using intracellular cAMP concentration as an indicator.

13. The method according to claim 12, wherein the intracellular cAMP concentration is measured by a reporter assay.

14. The method according to any one of claims 1 to 13, wherein the olfactory receptor is a human olfactory receptor.

15. The method according to any one of claims 1 to 14, wherein the OR5P3 is a protein described in the following (a), (b), or (c):

(a) a protein comprising the amino acid sequence shown in SEQ ID NO: 2;

(b) a protein comprising an amino acid sequence comprising substitution, deletion, insertion, and/or addition of 1 to 10 amino acid residues in the amino acid sequence shown in SEQ ID NO: 2, and having responsivity to the olfactory receptor-activating substance; or

(c) a protein comprising an amino acid sequence having an identity of 80% or higher to the amino acid sequence shown in SEQ ID NO: 2, and having responsivity to the olfactory receptor-activating substance.

16. The method according to any one of claims 1 to 15, wherein the olfactory receptor-activating substance is 4-methylacetophenone or 2-heptanone.

17. The method according to any one of claims 1 to 16, wherein the method further comprises the step of evaluating whether an identified substance that suppresses 4-methylacetophenone odor has a function to suppress 4-methylacetophenone odor.

18. The method according to claim 17, wherein the evaluation is performed by sensory evaluation.

19. A composition for suppressing 4-methylacetophenone odor of a food, comprising the following ingredient (A):
(A) at least one type of ingredient that inactivates the olfactory receptor OR5P3 and is selected from the group consisting of N-vanillylnonanamide, nootkatone, 2-phenylethanethiol, benzyl isothiocyanate, 2-thiophenemethanethiol, difurfuryl sulfide, 2-methylbenzenethiol, benzyl isoeugenol, 2-phenyl-2-butenal, 4-methyl-1-phenyl-2-pentanol, 2-thiophenethiol, 2,4-heptadienal, and S-(2-methyl-3-furyl) ethanethioate.

20. A composition for producing a food, comprising the following ingredient (A):
(A) at least one type of ingredient that inactivates the olfactory receptor OR5P3 and is selected from the group consisting of N-vanillylnonanamide, nootkatone, 2-phenylethanethiol, benzyl isothiocyanate, 2-thiophenemethanethiol, difurfuryl sulfide, 2-methylbenzenethiol, benzyl isoeugenol, 2-phenyl-2-butenal, 4-methyl-1-phenyl-2-pentanol, 2-thiophenethiol, 2,4-heptadienal, and S-(2-methyl-3-furyl) ethanethioate.

21. The composition according to claim 20, wherein the food is a food of which 4-methylacetophenone odor is suppressed.

22. The composition according to any one of claims 19 to 21, wherein the food comprises 4-methylacetophenone and/or an ingredient that can generate 4-methyl acetophenone.

23. The composition according to claim 22, wherein the ingredient that can generate 4-methylacetophenone is citral.

24. A method for suppressing 4-methylacetophenone odor of a food, comprising the step of adding the following ingredient (A) to a raw material of the food:
(A) at least one type of ingredient that inactivates the olfactory receptor OR5P3 and is selected from the group consisting of N-vanillylnonanamide, nootkatone, 2-phenylethanethiol, benzyl isothiocyanate, 2-thiophenemethanethiol, difurfuryl sulfide, 2-methylbenzenethiol, benzyl isoeugenol, 2-phenyl-2-butenal, 4-methyl-1-phenyl-2-pentanol, 2-thiophenethiol, 2,4-heptadienal, and S-(2-methyl-3-furyl) ethanethioate.

25. A method for producing a food, comprising the step of adding the following ingredient (A) to a raw material of the food:
(A) at least one type of ingredient that inactivates the olfactory receptor OR5P3 and is selected from the group consisting of N-vanillylnonanamide, nootkatone, 2-phenylethanethiol, benzyl isothiocyanate, 2-thiophenemethanethiol, difurfuryl sulfide, 2-methylbenzenethiol, benzyl isoeugenol, 2-phenyl-2-butenal, 4-methyl-1-phenyl-2-pentanol, 2-thiophenethiol, 2,4-heptadienal, and S-(2-methyl-3-furyl) ethanethioate.

26. The method according to claim 25, wherein the food is a food of which 4-methylacetophenone odor is suppressed.

27. The method according to any one of claims 24 to 26, wherein the food or the raw material comprises 4-methylacetophenone and/or an ingredient that can generate 4-methylacetophenone.

28. The method according to claim 27, wherein the ingredient that can generate 4-methylacetophenone is citral.

29. The method according to claim 27 or 28, wherein the method comprises the step of heating the food or the raw material comprising an ingredient that can generate 4-methylacetophenone.

30. The method according to any one of claims 24 to 29, wherein the ingredient (A) is added so that the concentration thereof at the time of eating is 0.001 ppt (w/w) to 100 ppm (w/w).

Fig. 1

Fig. 2

Fig. 3

## INTERNATIONAL SEARCH REPORT

International application No.

**PCT/JP2023/003134**

### A. CLASSIFICATION OF SUBJECT MATTER

*C12Q 1/06*(2006.01)i; *A23L 27/00*(2016.01)i; *C12N 15/12*(2006.01)i; *C12Q 1/6897*(2018.01)i
FI:   C12Q1/06 ZNA; C12Q1/6897 Z; A23L27/00 Z; C12N15/12

According to International Patent Classification (IPC) or to both national classification and IPC

### B. FIELDS SEARCHED

Minimum documentation searched (classification system followed by classification symbols)

C12Q1/06; A23L27/00; C12N15/12; C12Q1/6897

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

CAplus/MEDLINE/EMBASE/BIOSIS (STN)

### C. DOCUMENTS CONSIDERED TO BE RELEVANT

| Category* | Citation of document, with indication, where appropriate, of the relevant passages | Relevant to claim No. |
|---|---|---|
| X | WO 2021/193429 A1 (AJINOMOTO KK) 30 September 2021 (2021-09-30) claims | 20, 25, 30 |
| A | | 1-19, 21-24, 26-29 |
| X | JP 2019-129774 A (AJINOMOTO KK) 08 August 2019 (2019-08-08) claims | 20, 25, 30 |
| A | | 1-19, 21-24, 26-29 |
| A | JP 2019-129772 A (AJINOMOTO KK) 08 August 2019 (2019-08-08) claims | 1-30 |
| A | JP 2019-129773 A (AJINOMOTO KK) 08 August 2019 (2019-08-08) claims | 1-30 |
| A | JP 2021-136880 A (TOYOTA CENTRAL RES & DEV) 16 September 2021 (2021-09-16) claims | 1-30 |
| A | JP 2012-249614 A (KAO CORP) 20 December 2012 (2012-12-20) claims | 1-30 |

☑ Further documents are listed in the continuation of Box C.     ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **06 April 2023** | **18 April 2023** |

| Name and mailing address of the ISA/JP | Authorized officer |
|---|---|
| **Japan Patent Office (ISA/JP)** 3-4-3 Kasumigaseki, Chiyoda-ku, Tokyo 100-8915 **Japan** | |
| | Telephone No. |

Form PCT/ISA/210 (second sheet) (January 2015)

| | INTERNATIONAL SEARCH REPORT | International application No. |
|---|---|---|
| | | **PCT/JP2023/003134** |

**C.     DOCUMENTS CONSIDERED TO BE RELEVANT**

| Category* | Citation of document, with indication, where appropriate, of the relevant passages | Relevant to claim No. |
|---|---|---|
| A | JP 2012-250958 A (KAO CORP) 20 December 2012 (2012-12-20)<br>    claims | 1-30 |
| A | WO 2019/131789 A1 (TAKASAGO INTERNATIONAL CORPORATION) 04 July 2019<br>(2019-07-04)<br>    claims | 1-30 |
| A | JP 2018-530997 A (RESEARCH FOUNDATION OF THE CITY UNIVERSITY OF NEW<br>YORK) 25 October 2018 (2018-10-25)<br>    paragraph [0032] | 1-30 |
| A | US 2015/0038338 A1 (NEW YORK UNIVERSITY) 05 February 2015 (2015-02-05)<br>    example 9 | 1-30 |
| A | AUDOUZE, K. et al. Identification of Odorant-Receptor Interactions by Global Mapping of the<br>Human Odorome. PLOS ONE. 2014, vol. 9, no. 4, e93037, pp. 1-12<br>    abstract | 1-30 |
| P, A | JP 2022-25044 A (TAKASAGO INTERNATIONAL CORPORATION) 09 February 2022<br>(2022-02-09)<br>    claims | 1-30 |

Form PCT/ISA/210 (second sheet) (January 2015)

**INTERNATIONAL SEARCH REPORT**

International application No.

**PCT/JP2023/003134**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a. ☑ forming part of the international application as filed:

        ☑ in the form of an Annex C/ST.25 text file.

        ☐ on paper or in the form of an image file.

    b. ☐ furnished together with the international application under PCT Rule 13*ter*.1(a) for the purposes of international search only in the form of an Annex C/ST.25 text file.

    c. ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

    "In the form of an Annex C/ST.25 text file" above should be understood as "in ST.26 format".

Form PCT/ISA/210 (continuation of first sheet) (January 2015)

INTERNATIONAL SEARCH REPORT
Information on patent family members

International application No.

**PCT/JP2023/003134**

| Patent document cited in search report | | | Publication date (day/month/year) | Patent family member(s) | | | Publication date (day/month/year) |
|---|---|---|---|---|---|---|---|
| WO | 2021/193429 | A1 | 30 September 2021 | CN | 115334906 | A | |
| JP | 2019-129774 | A | 08 August 2019 | (Family: none) | | | |
| JP | 2019-129772 | A | 08 August 2019 | (Family: none) | | | |
| JP | 2019-129773 | A | 08 August 2019 | (Family: none) | | | |
| JP | 2021-136880 | A | 16 September 2021 | (Family: none) | | | |
| JP | 2012-249614 | A | 20 December 2012 | US claims WO EP | 2014/0186864 2012/169644 2718718 | A1 A1 A1 | |
| JP | 2012-250958 | A | 20 December 2012 | (Family: none) | | | |
| WO | 2019/131789 | A1 | 04 July 2019 | US claims EP | 2020/0318205 3733862 | A1 A1 | |
| JP | 2018-530997 | A | 25 October 2018 | US paragraph [0037] WO EP CN | 2018/0317465 2017/024028 3331990 108138131 | A1 A1 A1 A | |
| US | 2015/0038338 | A1 | 05 February 2015 | (Family: none) | | | |
| JP | 2022-25044 | A | 09 February 2022 | US claims EP | 2022/0034866 3944854 | A1 A2 | |

Form PCT/ISA/210 (patent family annex) (January 2015)

## REFERENCES CITED IN THE DESCRIPTION

### Patent documents cited in the description

- JP 2015180715 A
- JP 2002338990 A
- JP 2008280539 A
- JP 2019129772 A
- WO 2005000558 A
- US 20180095071 A1
- JP 2019037197 A

### Non-patent literature cited in the description

- **WATANABE R. et al.** Arrayed lipid bilayer chambers allow single-molecule analysis of membrane transporter activity. *Nat Commun.*, 24 July 2014, vol. 5, 4519
- **KAMIYA K. et al.** Preparation of artificial cell membrane and single ion channel measurement. *Electrochemistry*, 2015, vol. 83, 1096-1100
- **KAWANO R. et al.** Automated Parallel Recordings of Topologically Identified Single Ion Channels. *Scientific Reports*, 2013, vol. 3 (1995)
- *Gene,* 1987, vol. 60 (1), 115-127
- **HIGUCHI, R.** PCR technology. Stockton press, 1989
- **CARTER, P.** *Meth. in Enzymol.*, 1987, vol. 154, 382
- **KRAMER, W.** ; **FRITS, H.J.** *Meth. in Enzymol.*, 1987, vol. 154, 350
- **KUNKEL, T.A. et al.** *Meth. in Enzymol.*, 1987, vol. 154, 367
- **GOLDSTEIN et al.** Prokaryotic promoters in biotechnology. *Biotechnol. Annu. Rev.*, 1995, vol. 1, 105-128
- **ZHUANG H.** ; **MATSUNAMI H.** *J. Biol. Chem.*, 2007, vol. 282, 15284-15293
- **KAJIYA K. et al.** Molecular bases of odor discrimination: Reconstitution of olfactory receptors that recognize overlapping sets of odorants. *Journal of Neuroscience*, 2001, vol. 21, 6018-6025
- **K. KAJIYA et al.** *Journal of Neuroscience*, 15 August 2001, vol. 21 (16), 6018-6025